# EUROPEAN PATENT SPECIFICATION

(11) **EP 4 142 728 B1**
(45) Date of publication and mention of the grant of the patent: **29.01.2025**
(21) Application number: 21723639.7
(22) Date of filing: 26.04.2021
(51) Int. Cl.: A61K 31/49, A23L 33/00, A61K 9/00, A61P 31/14

(54) **6'-METHOXYCINCHONAN-9-OLS FOR THE TREATMENT OF CORONAVIRAL INFECTIONS**
6'-METHOXYCINCHONAN-9-OLE ZUR BEHANDLUNG VON CORONAVIRUS-INFEKTIONEN
6'-MÉTHOXYCINCHONAN-9-OLS POUR LE TRAITEMENT D'INFECTIONS À CORONAVIRUS

(30) Priority: 27.04.2020 EP 20000170; 28.04.2020 EP 20000172
(43) Date of publication of application: 08.03.2023
(73) Proprietor: Immunologik GmbH, 13507 Berlin (DE)
(72) Inventor: SETZ, Christian, 96117 Memmelsdorf (DE); SCHUBERT, Ulrich, 07646 Trockenborn-Wolfersdorf (DE); RHEBER, Sascha, 07616 Thalbürgel (DE)
(74) Representative: Gruber, Daniel
(86) International application number: PCT/EP2021/000051
(87) International publication number: WO 2021/219244

(56) References cited:
- WO-A1-03/039546
- WO-A1-2021/216759
- ANONYMOUS: "History of Changes for Study: NCT04408183 GLS-1200 Topical Nasal Spray to Prevent SARS-CoV-2 Infection (COVID19) in Health Care Personnel", CLINICALTRIALS.GOV ARCHIVE, 24 March 2021 (2021-03-24), pages 1 - 7, XP093117416, Retrieved from the Internet <URL:https://classic.clinicaltrials.gov/ct2/history/NCT04408183?V_4=View#StudyPageTop> [retrieved on 20240109]
- LI XIANGQI ET AL: "Existing bitter medicines for fighting 2019-nCoV-associated infectious diseases", THE FASEB JOURNAL, FEDERATION OF AMERICAN SOCIETIES FOR EXPERIMENTAL BIOLOGY, UNITED STATES, vol. 34, no. 5, 30 April 2020 (2020-04-30), pages 6008 - 6016, XP009526071, ISSN: 1530-6860, DOI: 10.1096/FJ.202000502
- ESTARI MAMIDALA ET AL: "In silico identification of clinically approved medicines against the main protease of SARS-CoV-2, causative agent of covid-19", ARXIV.ORG, CORNELL UNIVERSITY LIBRARY, 201 OLIN LIBRARY CORNELL UNIVERSITY ITHACA, NY 14853, 25 April 2020 (2020-04-25), XP081652803
- TOURET FRANCK ET AL: "In vitro screening of a FDA approved chemical library reveals potential inhibitors of SARS-CoV-2 replication", 5 April 2020 (2020-04-05), XP055813369, Retrieved from the Internet <URL:https://www.biorxiv.org/content/10.1101/2020.04.03.023846v1.full.pdf> [retrieved on 20210614], DOI: 10.1101/2020.04.03.023846
- ONG C M Y ET AL: "Permeation of quinine across sublingual mucosa, in vitro", INTERNATIONAL JOURNAL OF PHARMACEUTICS, ELSEVIER, NL, vol. 366, no. 1-2, 21 January 2009 (2009-01-21), pages 58 - 64, XP025839899, ISSN: 0378-5173, [retrieved on 20080912], DOI: 10.1016/J.IJPHARM.2008.08.048
- MISCHEL FIONA: "What You Should Know About Drinks and Foods That Contain Quinine | Livestrong.com", LIVESTRONG.COM, 30 March 2020 (2020-03-30), pages 1 - 14, XP055819973, Retrieved from the Internet <URL:https://www.livestrong.com/article/304030-list-of-foods-that-contain-quinine/> [retrieved on 20210630]
- A SUMITHA ET AL: "Covid-19 - In Silico Structure Prediction and Molecular Docking Studies with Doxycycline and Quinine", BIOMEDICAL & PHARMACOLOGY JOURNAL, vol. 13, no. 03, 11 September 2020 (2020-09-11), IN, pages 1185 - 1193, XP055815264, ISSN: 0974-6242, Retrieved from the Internet <URL:https://biomedpharmajournal.org/vol13no3/covid-19-in-silico-structure-prediction-and-molecular-docking-studies-with-doxycycline-and-quinine/> DOI: 10.13005/bpj/1986
- GENDROT MATHIEU ET AL: "Antimalarial drugs inhibit the replication of SARS-CoV-2: An in vitro evaluation", TRAVEL MEDICINE AND INFECTIOUS DISEASE, ELSEVIER, AMSTERDAM, NL, vol. 37, 1 September 2020 (2020-09-01), XP086301519, ISSN: 1477-8939, [retrieved on 20200908], DOI: 10.1016/J.TMAID.2020.101873
- GROSSE MAXIMILIAN ET AL: "Quinine Inhibits Infection of Human Cell Lines with SARS-CoV-2", VIRUSES, vol. 13, no. 4, 9 April 2021 (2021-04-09), pages 647, XP055819928, Retrieved from the Internet <URL:https://www.ncbi.nlm.nih.gov/pmc/articles/PMC8069458/pdf/viruses-13-00647.pdf> DOI: 10.3390/v13040647

## Description

The present application relates to 6'-methoxycinchonan-9-ols for use in the prevention or treatment of a coronaviral infection, as defined in the claims.

Pharmaceutical compositions, combinations with antiviral agents, advantageous formulation techniques and a method of treatment are disclosed. Compositions of dietary supplements containing quinine are provided.

### BACKGROUND OF THE INVENTION

As a result of ecological, climatic and demographic changes, so-called 'emerging' viruses are increasingly being transmitted from their natural animal hosts to humans. Due to accelerated globalization they bear the risk of triggering a pandemic. Emerging viruses may cause acute and often life-threatening diseases. *Coronaviridae* have become notorious for such transmissions. Examples are *Severe acute respiratory syndrome coronavirus* (SARS-CoV) and *Middle East respiratory syndrome-related coronavirus* (MERS-CoV), and most recently, the outbreak of *Severe acute respiratory syndrome coronavirus 2* (SARS-CoV-2; COVID-19) in Wuhan, China. By April 22^{nd}, 2021 a total of worldwide over 143,874,630 SARS-CoV-2 cases with over 3 million fatalities have been reported by Johns Hopkins University. The incubation period of SARS-CoV-2 ranges between two days and one month.

Typical symptoms of SARS-CoV-2 are fever, cough, and shortness of breath, as well as neurological symptoms such as loss of smell or taste. However, the infection can also cause severe pulmonary injury, leading to rapid onset of progressive malfunction of the lungs, especially with regard to the ability to take up oxygen. This is usually associated with the malfunction of other organs. This acute lung injury (ALI) condition is associated with extensive lung inflammation and accumulation of fluid in the alveoli. It is characterized by diffuse pulmonary microvascular injury resulting in increased permeability and, thus, non-cardiogenic pulmonary edema. In consequence, this leads to pathologically low oxygen levels in the lungs.

Coronaviruses are primarily spread through close contact, in particular through respiratory droplets from coughs and sneezes. In contrast to SARS-CoV and MERS-CoV, SARS-CoV-2 can be transmitted from human to human during the incubation period while the infected patient does not show yet any symptoms of disease. Moreover, SARS-CoV-2 can already replicate in the throat. In contrast, the receptors for SARS -CoV and MERS-CoV are located deep in the lungs. Thus, SARS-CoV-2 can be transmitted much easier from human to human in comparison to SARS-CoV and MERS-CoV which strongly increases the infection rate.

In general, coronaviruses (family Coronaviridae, group Coronaviruses) form a relatively diverse group of large, enveloped, positive strand RNA viruses, which can cause different types of diarrhea and respiratory diseases in humans and animals. They have a very narrow host range and replicate very poorly in cell culture. However, cell culture systems for SARS-CoV-2 could be successfully established.

Sequencing of SARS-CoV-2 revealed an approx. 29.8 kbp genome consisting of 14 open reading frame. Moreover, the virus is phylogenetically closely related to the SARS-CoV (89.1 % nucleotide similarity) (cf. Wu et al. (2020) Nature*,* Epub ahead of print). Like other coronaviruses, SARS-CoV-2 enters the cell by endocytosis and membrane fusion. The viruses are released from the cell by the secretory pathway. The natural reservoir of the virus is unknown.

To date, no specific therapeutic options for the treatment of SARS-CoV-2 infections, respectively COVID-19 are established. Some success could be achieved with the antiviral drugs remdesivir, avifavir and favipiravir as well as with the anti-parasitic drug ivermectin. A nasal spray containing nanoantibodies against the SARS-CoV-2 spike protein is a promising development (AeroNabs). In severe stage COVID-19 patients the administration of the glucocorticoid dexamethasone showed to be effective. Also the inhalable glucocorticoid budesonide may keep a SARS-CoV-2 infection in a non-severe stage.

WO 03/039546 discloses the use of anti-malarial compounds, among them quinine and quinidine, for the prophylaxis and treatment of viral respiratory diseases such as human corona virus infection; SARS-CoV-2 is not mentioned.

In Mamidala et al. (2020, Cornell University Library, 201 OLIN library) *in silico* calculations of the potential inhibitory activity of various compounds on the main protease of SARS-CoV-2 are described. Among the identified candidates is quinidine, but not quinine.

Touret et al. (2020, bioRxiv preprint doi: https.//doi.org/10.1101/2020.04.03023846) disclose that quinidine inhibits *in vitro* SARS-CoV-2 replication.

Sumitha et al. (2020; Biomed Pharmacol J 13: 1185-1193; postpublished) predict based on *in silico* structures and molecular docking studies an effect of doxycycline and quinine in the treatment of COVID-19.

Gendrot et al. (2020; Travel Med Infect Dis 37: 101873; postpublished) reveal that amtimalarial drugs, among them quinine, inhibit SARS-CoV-2 replication *in vitro.*

In WO 2021/216759 it is described how quinine and other bitter taste receptor agonists activate the innate immune response against viral upper respiratory infections, among them SARS-CoV-2. A prophylaxis and treatment by applying these bitter taste receptor agonists on the mucosal surface of an upper respiratory cavity of a subject is proposed.

In the clinical study NCT04408183 (on ClinicalTrials.gov; postpublished) it was shown that a topical nasal spray containing quinine is able to prevent SARS-CoV-2 infection in health care personnel.

Xiangqi et al. (first published on 13.04.2020, FASEB J., 34(5), 6008-6016) disclose that agonists of type 2 taste receptors (TAS2Rs), such as quinine, may target the most common symptoms caused by 2019-nCoV. It is suggested that a cocktail-like recipe of existing bitter drugs may help to fight this disease.

Thus, there is a strong medical need for an effective pharmacological treatment for patients infected with SARS-CoV-2 or similar coronaviruses and for limiting the epidemic spread of this virus. Ideally, such a pharmacological treatment should also offer at least a treatment option for future coronavirus outbreaks.

Surprisingly, this task is solved by the 6'-methoxycinchonan-9-ols according to the disclosure.

### DESCRIPTION OF THE INVENTION

The present invention relates to quinine or one of its pharmaceutically acceptable salts, hydrates and solvates for use by oral or pharyngeal administration in the prophylaxis or treatment of a coronaviral infection caused by SARS-CoV-2. The disclosures relating to quinidine are for reference and are not part of the claimed invention.

There are two diastereomers of 6'-methoxycinchonan-9-ol: Quinine ( (-)-(8*α*,9*R*)-6'-methoxycinchonan-9-ol; synonyms: 1-(6-methoxyquinoline-4-yl)-1-(5-vinyl-1-azabicyclo[2.2.2]oct-2-yl)methanol, 1-(6-methoxyquinoline-4-yl)-1-(5-vinyl-1,4-ethanopiperidine-2-yl)methanol ) and quinidine ( (+)-(9*S*)-6'-methoxycinchonan-9-ol; synonym: (2-ethenyl-4-azabicyclo[2.2.2]oct-5-yl)-(6-methoxyquinoline-4-yl)-methanol ).

Thus, in the scope of the present disclosure the terms "6'-methoxycinchonan-9-ols" and "a 6'-methoxycinchonan-9-ol" shall refer to quinine and quinidine. Only quinine is part of the claimed invention.

Quinine and quinidine are quinoline alkaloids. Traditionally, quinine was obtained from quina bark (*Cinchona pubescens,* a rubiacea growing in high altitude forests in South America). Quinine is a white, poorly water-soluble crystalline powder with a bitter taste.

It was used long time in folk medicine for treating febrifugal diseases. Quinine was the first drug found to be effective in the treatment of malaria, in particular of the complicated form Malaria tropica. It complexes toxic ferriprotoporphyrin IX and thus inhibits the formation of non-toxic beta-haematin in the vacuoles of blood schizonts of *Plasmodium falciparum.* Nowadays it is still in use in the treatment of chloroquine-refractory malaria pathogens and if artemisinins are not indicated. In the USA it is often used against Malaria tropica. It is further used against babesiosis (*Babesia* infections).

Quinine has also analgesic, local anesthetic and antipyretic properties. Therefore, low doses of quinine are used in China for treating common colds.

When administered in low doses as quinine sulfate it also used to resolve cramps such as leg cramps at night.

It has also been in use for treating restless leg syndrome but was discontinued because of side effects.

Further indications are lupus erythematosus, osteoarthrosis and rheumatoid arthritis.

Adverse side effects include thrombocytopenia, thrombotic microangiopathy and methemoglobinemia. Long-term therapy with high doses of quinine sulfate may lead to nausea, headache, sweating, tinnitus, visual disturbances, fever, hypotension, hemolytic anemia, acute kidney injury, liver toxicity, blindness and disorders of the gastrointestinal tract, the dermis, the cardiovascular system (low platelet count, hemolytic-uremic syndrome/thrombotic thrombocytopenic purpura (HUS/TTP), long QT syndrome and other serious cardiac arrhythmias including torsades de pointes, blackwater fever, disseminated intravascular coagulation, leukopenia, neutropenia) and the nervous system, in rare cases asthma, hemoglobinemia (cf. Liles et al. (2016) Am J Hematol 91: 461-466).

In the food industry, quinine is used as a bitter flavoring. It can be added to fancy drinks like bitter lemon or tonic water (in the European Union up to 100 mg/kg, in Germany 85 mg/kg) or to alcoholic beverages like gin tonic and bitters (in the European Union up to 300 mg/kg, in Germany 250 mg/kg).

As shown in Examples 1 to 3, quinine is able to block dose-dependently and effectively the replication of SARS-CoV-2 in infected Vero-B4 cells at non-toxic concentrations.

Quinidine was the first antiarrhythmic agent that was found to be effective. It can be administered intravenously or orally. It is classified as a class 1A antiarrhythmic, as it binds to open sodium channels (in particular Naᵥ1.5). There is frequency-dependency of its action (use-dependent block), as the quinidine / ion channel complex only slowly dissociates. Quinidine decreases also the potassium conductivity (in particular Kᵥ1.4, Kᵥ4.2, Kᵥ11.1), leading to increased cardiac action potential duration and a prolonged QT interval. Moreover, quinidine blocks cardiac calcium channels in an atropine-like manner and is an alpha-1 blocker (Shibata et al. (1998) Circulation 97: 1227-1230). Quinidine is thus used in the treatment of atrial fibrillation, extrasystoles and ventricular tachyarrhythmias.

Seldomly, intravenously administered quinidine is used against *Plasmodium falciparum* malaria.

Because of serious adverse side effects it is nowadays seldomly used. These effects include long QT syndrome, atrioventricular block, torsade de pointes arrhythmias, ventricular tachycardias as well as gastrointestinal disorders). When administered intravenously, quinidine strongly reduces vascular resistance. Further adverse side effects include thrombocytopenia (eventually leading to thrombocytic purpura), granulomatous hepatitis and myasthenia gravis.

Further, quinidine inhibits cytochrome P450 enzyme 2D6 and thus increases the blood level of a number of medications.

As shown in Examples 4 to 6, quinidine (not part of the claimed invention) is able to block dose-dependently and effectively the replication of SARS-CoV-2 in infected Vero-B4 cells at non-toxic concentrations. Pharmaceutically acceptable salts should be seen in terms of this disclosure as an active agent containing a compound according to the disclosure in form of a salt, in particular if this salt bestows specific or ameliorated pharmacokinetic properties in comparison to the free form of the active agent or to another salt of the active agent. The pharmaceutically acceptable salt of the active agent may also bestow a pharmacokinetic characteristic to the active agent it did not have in its free form. Thus, it may even positively influence the pharmacodynamics of the active agent in respect to its therapeutic efficacy in the organism. The 6'-methoxycinchonan-9-ols according to the disclosure can be provided as pharmaceutically acceptable salts of organic and inorganic acids. Suitable examples are hydrochloric acid, hydrobromic acid, sulfuric acid, phosphoric acid, carbonic acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, digluconic acid, lactic acid, tartaric acid, hydroxymaleic acid, pyruvic acid, phenylacetic acid, benzoic acid, p-aminobenzoic acid, p-hydroxybenzoic acid, dinitrobenzoic acid, chlorbenzoic acid, methanesulfonic acid, ethanesulfonic acid, nitric acid, hydroxyethanesulfonic acid, ethylenesulfonic acid, phenolsulfonic acid, p-toluylsulfonic acid, naphthylsulfonic acid, sulfanilic acid, camphorsulfonic acid, alginic acid, capric acid, hippuric acid, pectinic acid, phthalic acid, quinic acid, pidolic acid, mandelic acid, o-methyl mandelic acid, hydrogen benzenesulfonic acid, picric acid, adipic acid, cyclopentane propionic acid, D-o-toluyl tartaric acid, tartronic acid, benzenesulfonic acid, alpha-methyl benzoic acid, (o, m, p-)methyl benzoic acid, naphthylamine sulfonic acid, phytic acid, tannic acid, boric acid, silicic acid, stannic acid, titanic acid as well as salts from other mineral acids or carbonic acids well known to a person skilled in the art. These salts are generated by contacting the free base with a sufficient amount of the respective acid in order to build the salt in a conventional manner.

Known salts of quinine include quinine hydrochloride, quinine dihydrochloride, quinine sulfate, quinine bisulfate, quinine sulfate dihydrate [(quinine)₂H₂SO₄·H₂O], quinine gluconate.

Known salts of quinidine include quinidine sulfate dihydrate [(quinidine)₂H₂SO₄·H₂O], quinidine sulfate, quinidine hydrochloride monohydrate.

These known salts of quinine and quinidine are preferred.

The compounds according to the disclosure can also be provided as hydrates or solvates. In terms of this disclosure solvates refer to such forms of the compounds according to the disclosure that build a complex through coordination with solvent molecules. Hydrates are a specific form of solvates in which the coordination is effected by water molecules.

Thus, the present application relates to 6'-methoxycinchonan-9-ols as well as their pharmaceutically acceptable salts, hydrates and solvates for use in the prophylaxis or treatment of a coronaviral infection.

In particular, the present application relates to quinine (according to the invention) and/or quinidine (not part of the claimed invention) as well as one of its pharmaceutically acceptable salts, hydrates or solvates for use in the prophylaxis or treatment of a coronaviral infection.

The coronaviral infections that can be treated with a 6'-methoxycinchonan-9-ol according to the disclosure are above all infections with the highly pathogenic SARS-CoV; MERS-CoV and SARS-CoV-2. But also infections with less pathogenic coronaviridae as listed in the following can be thus treated. The invention relates to the prophylaxis or treatment of a coronaviral infection caused by SARS-CoV-2, as defined in the claims.

The terms "coronavirus" or "coronaviral" refer mainly to the sub-familiy of orthocoronavirinae. They are subdivided into the genera of alphacoronaviruses, betacoronaviruses, gammacoronaviruses and deltacoronaviruses. Alphacoronaviruses comprise the sub-genera of colacoviruses (species: bat coronavirus CDPHE15), decaviruses (bat coronavirus HKU10, Rhinolophus ferrumequinum alphacoronavirus HuB-2013), duvinacoviruses (human coronavirus 229E), luchacoviruses (Lucheng Rn rat coronavirus), minacoviruses (Ferret coronavirus, Mink coronavirus 1), minunacoviruses (miniopterus bat coronavirus 1, miniopterus bat coronavirus HKU8), myotacoviruses (Myotis ricketti alphacoronavirus Sax-2011), nylactoviruses (Nyctalus velutinus alphacoronavirus SC-2013), pedacoviruses (porcine epidemic diarrhea virus, Scotophilus bat coronavirus 512), rhinacoviruses (Rhinolophus bat coronavirus HKU2), setracoviruses (human coronavirus NL63, NL63-related bat coronavirus strain BtKYNM63-9b) and tegacoviruses (Alphacoronavirus 1 - type species). Betacoronaviruses comprise the sub-genera of embecoviruses (Betacoronavirus 1 (subspecies: human coronavirus OC43), China Rattus coronavirus HKU24, human coronavirus HKU1, murine coronavirus-type species), hibecoviruses (Bat Hp-betacoronavirus Zhejiang 2013), merbecoviruses (Hedgehog coronavirus 1, MERS-CoV), Pipistrellus bat coronavirus HKU5, Tylonycteris bat coronavirus HKU4), nobecoviruses (Rousettus bat coronavirus GCCDC1, Rousettus bat coronavirus HKU9 and sarbecoviruses (severe acute respiratory syndrome-related coronavirus (subspecies: SARS-CoV, SARS-CoV-2). Gammacoronaviruses comprise the sub-genera of cegacoviruses (Beluga whale coronavirus SW1) and igacoviruses (Avian coronavirus-type species). Deltacoronaviruses comprise the sub-genera of andecoviruses (Wigeon coronavirus HKU20), buldecoviruses (Bulbul coronavirus HKU11-type species, Porcine coronavirus HKU15, Munia coronavirus HKU13, White-eye coronavirus HKU16), herdecoviruses (Night heron coronavirus HKU19) and Moordecoviruses (Common moorhen coronavirus HKU21).

Coronaviruses pathogenic in humans are until now SARS-CoV, SARS-CoV-2, MERS-CoV and HCoV-HKU1, HCoV-NL-63, HCoV-OC43 and HCoV-229E. The last four cause only relatively mild symptoms (cf. Andersen et al.: The Proximal Origin of SARS-CoV-2 (2020) Nat Med 26: 450-452).

Thus, the present application relates in particular to 6'-methoxycinchonan-9-ols for use according to the disclosure, wherein said coronaviral infection is selected from a group comprising a SARS-CoV, SARS-CoV-2, MERS-CoV, HCoV-HKU1, HCoV-NL-63, HCoV-OC43 and HCoV-229E infection.

Preferentially, quinine or quinidine are used in the prevention or therapy of SARS-CoV, SARS-CoV-2 or MERS-CoV infections. Most preferred quinine or quinidine are used in the prevention or therapy of SARS-CoV-2 infections. The invention relates to quinine or one of its pharmaceutically acceptable salts, hydrates and solvates for use by oral or pharyngeal administration in the prophylaxis or treatment of a coronaviral infection caused by SARS-CoV-2.

The other above-mentioned animal coronaviruses have not yet made a transfer to humans (zoonosis), but this may happen in the future with an unpredictable pathology. Thus, the present application relates also to 6'-methoxycinchonan-9-ols according to the disclosure for the treatment of these animal coronaviral infections in animals and humans. The concept for treating coronaviral infections over all species is based on the structural similarity of coronaviruses. Thus, it can be assumed that treatment and/or prevention options can be transferred from one coronavirus to another. Coronavirus particles contain four main structural proteins: spike (S), membrane (M), envelope (E) and nucleocapsid (N), all of which are encoded within the 3' end of the viral genome.

Coronaviruses contain a non-segmented, positive-sense RNA genome of ~30 kb. The genome contains a 5' cap structure along with a 3' poly (A) tail, allowing it to act as an mRNA for translation of the replicase polyproteins. The replicase gene encoding the nonstructural proteins (nsps) occupies two-thirds of the genome, about 20 kb, as opposed to the structural and accessory proteins, which make up only about 10 kb of the viral genome. The organization of the coronavirus genome is 5'-leader-UTR- replicase-S (Spike)-E (Envelope)-M (Membrane)- N (Nucleocapsid)-3' UTR-poly (A) tail with accessory genes interspersed within the structural genes at the 3' end of the genome. The accessory proteins are almost exclusively nonessential for replication in tissue culture; however, some have been shown to have important roles in viral pathogenesis (cf. Zhao et al. (2012) Cell Host Microbe 11: 607-616).

The coronavirus life cycle starts with an initial attachment of the virion to the host cell by interactions between the S protein and its receptor. The sites of receptor binding domains (RBD) within the S1 region of a coronavirus S protein vary depending on the virus. The S-protein-receptor interaction is the primary determinant for a coronavirus to infect a host species and also governs the tissue tropism of the virus. Many coronaviruses utilize peptidases as their cellular receptor. It is unclear why peptidases are used, as entry occurs even in the absence of the enzymatic domain of these proteins. Many alphacoronaviruses utilize aminopeptidase N (APN) as their receptor, many betacoronaviruses such as SARS-CoV, SARS-CoV-2 and HCoV-NL63 use angiotensin-converting enzyme 2 (ACE2) as its receptor for cell entry, MHV enters through CEACAM1, and MERS-CoV binds to dipeptidyl-peptidase 4 (DPP4) to gain entry into human cells. Following receptor binding, the virus must next gain access to the host cell cytosol. This is generally accomplished by acid-dependent proteolytic cleavage of S protein by a cathepsin, TMPRRS2 or another protease, followed by fusion of the viral and cellular membranes, and ultimately the release of the viral genome into the cytoplasm.

Coronaviruses encode either two or three proteases that cleave the replicase polyproteins. They are the papain-like proteases (PLpro), encoded within nsp3, and a serine type protease, the main protease, or Mpro, encoded by nsp5. Most coronaviruses encode two PLpros within nsp3, except the gammacoronaviruses, SARS-CoV and MERS-CoV, which only express one PLpro (Mielech et al. (2014) Virus Res 194: 184-190).

This papain-like protease (PLpro) was found in SARS-CoV to act the same way as a deubiquitinase within the human cellular ubiquitin proteasome system (UPS) (cf. Raaben et al. (2010) J Virol 84: 7869-7879). PLpro in SARS-CoV-2 has a very high homology with SARS-CoV (96.1 %, Nguyen et al. (2020) PLoS One 15: e0242537).

For an effective treatment of coronaviral infections it may be advantageous to provide to a patient in need thereof a combinational therapy by combining a 6'-methoxycinchonan-9-ol according to the disclosure with at least one antiviral agent.

For example, from HIV, respectively anti-retroviral therapy the following classes are known:
Reverse transcriptase inhibitors suitable for such a combination therapy are nucleoside reverse transcriptase inhibitors (NRTI) and non-nucleoside reverse transcriptase inhibitors (NNRTI). Examples of NRTI include, but are not limited to, abacavir, didanosine, emtricitabine, lamivudine, stavudine, tenofovir, zidovudine, zalcitabine, entecavir, adefovir, elvucitabine, fosalvudine(-tidoxil), fozivudintidoxil, lagiciclovir, alamifovir, clevudine, pradefovir, telbivudine. Examples of NNRTI include, but are not limited to, efavirenz, etravirine, nevirapine, rilpivirine, delavirdine, emivirine, lersivirine.

Suitable for a combination therapy according to the disclosure are integrase inhibitors such as raltegravir, elvitegravir, dolutegravir, MK-2048.

Examples of HIV protease inhibitors suitable for a combination therapy according to the disclosure are saquinavir, indinavir, ritonavir, nelfinavir, amprenavir, lopinavir, atazanavir, fosamprenavir, tipranavir, darunavir, brecanavir, mozenavir, tipranavir.

Examples of entry inhibitors suitable for a combination therapy according to the disclosure are enfuvirtide and maraviroc.

Further, general virostatic agents suitable for a combination therapy according to the disclosure can be selected from the group comprising ancriviroc, aplaviroc, cenicriviroc, enfuvirtide, maraviroc, vicriviroc, amantadine, rimantadine, pleconaril, idoxuridine, aciclovir, brivudine, famciclovir, penciclovir, sorivudine, valaciclovir, cidofovir, ganciclovir, valganciclovir, sofosbusvir, foscarnet, ribavirine, taribavirine, filibuvir, nesbuvir, tegobuvir, fosdevirine, favipiravir, merimepodib, asunaprevir, balapiravir, boceprivir, ciluprevir, danoprevir, daclatasvir, narlaprevir, telaprevir, simeprevir, vanipevir, rupintrivir, remdesivir, fomivirsen, amenamevir, alisporivir, bevirimat, letermovir, laninamavir, oseltamivir, peramivir, zanamivir.

General immunostimulatory agents suitable for a combination therapy according to the disclosure can be selected from the group comprising interferons (alpha-, beta-, gamma-, tau- interferon), interleukins, CSF, PDGF, EGF, IGF, THF, levamisol, dimepranol, inosine.

Furthermore, possible combinations according to the disclosure include adjuvants such as cobicistat.

The terms "medicine" or "medical" comprise human as well as veterinary medicine.

The term "organism" refers to a living being, especially a human or an animal, possessing a self-regulating immunological system.

The term "host organism" is used in terms of the application for those organisms exploited for replication by viruses, herein especially retroviruses, following an infection with them.

The term "active agent" in this application refers to 6'-methoxycinchonan-9-ol according to the disclosure. Moreover, this term can comprise further pharmaceutical agents, known from the state of the art.

The terms "composition" and "pharmaceutical composition" comprise at least one 6'-methoxycinchonan-9-ol according to the disclosure in any pharmacologically suitable defined dose and dosage form together with at least one suitable excipient or carrier substance as well as all substances which are directly or indirectly generated as a combination, accumulation, complex formation or crystal of the aforementioned ingredients, or come into being as a result of other reactions or interactions as well as optionally at least one further pharmaceutical agent known in the state of the art.

The term "excipient" is used in this application to describe each component of
a pharmaceutical composition in addition to the active agent. The selection of a suitable excipient depends on factors such as dosage form and dose as well as the influence on the solubility and stability of the composition by the excipient itself.

The term "action" describes the inherent specific mode of action of the respective agent in the scope of the present application.

The terms "effect", "therapeutic effect", "action", "therapeutic action" regarding at least one active agent according to the disclosure refer to causally occurring beneficial consequences for the organism, to which the at least one active agent has been administered.

In terms of the application, "therapeutically effective dose" means that a sufficient dose of the at least one 6'-methoxycinchonan-9-ol according to the disclosure is administered to a living being or to a patient in need of such a treatment.

The terms "joint administration", "combined administration" or "simultaneous administration" of at least one pharmaceutical agent according to the disclosure and/or of at least one pharmaceutical agent from the state of the art comprise the administration of the mentioned agents at the same time or at time points factually related close to each other, as well as administrations of said agents at different times within a coherent experiment. The chronological order of the administration of said agents is not limited by these terms. Those skilled in the art will have no difficulties to deduce the described administrations in respect to their chronological or local order from his knowledge and experience.

The term "living being" refers to every animal, especially vertebrate, including human. A "patient" in terms of the application is a living being who suffers from a definable and diagnosable disease, and to whom a suitable active agent can be administered.

The terms "prophylaxis", "treatment" and "therapy" comprise the administration of a 6'-methoxycinchonan-9-ol according to the disclosure alone or in combination with at least one further pharmaceutical agent known in the art, to a living being, in order to prevent the development of a certain disease, to inhibit, and to alleviate the symptoms, or to initiate a healing process of the respective disease.

The at least one 6'-methoxycinchonan-9-ol according to the disclosure can be applied in the prophylaxis and/or treatment of coronaviral infections by any medically acceptable administration route to a patient in need thereof. Such medically acceptable administration routes can be e.g. by inhalation, by intubation, orally, parenterally, intraperitoneally, intravenously, intraarterially, intramuscularly, topically, transdermally, subcutaneously, intradermally, sublingually, conjunctivally, intravaginally, rectally or nasally. According to the invention, quinine or one of its pharmaceutically acceptable salts, hydrates and solvates is administered by oral or pharyngeal administration.

In another aspect of the disclosure a pharmaceutical composition for use in the prophylaxis or treatment of coronaviral infections is disclosed, wherein said composition comprises at least one 6'-methoxycinchonan-9-ol according to the disclosure, a carrier and at least one pharmaceutically acceptable excipient.

The term "pharmaceutically acceptable excipient(s)" refers to natural or synthetic compounds that are added to a pharmaceutical formulation alongside the pharmaceutical active agent. They may help to bulk up the formulation, to enhance the desired pharmacokinetic properties or the stability of the formulation, as well as being beneficial in the manufacturing process. Advantageous classes of excipients according to the disclosure include, carriers, binding agents, colorants, buffers, preservatives, antioxidants, coatings, sweeteners, thickening agents, pH-regulators, acidity regulators acidifiers, solvents, isotonizing agents, penetration enhancers, disintegrants, glidants, lubricants, emulsifiers, solubilizing agents, stabilizers, diluents, anti-caking agents (antiadherents), sorbents, foaming agents, anti-foaming agents, opacifiers, fatliquors, consistency enhancers, hydrotropes, aromatic and flavoring substances.

In general, one or more pharmaceutically acceptable carriers are added to a pharmaceutically active agent. Eligible are all carriers known in the art and combinations thereof. In solid dosage forms they can be for example plant and animal fats, waxes, paraffins, starch, tragacanth, cellulose derivatives, polyethylene glycols, silicones, bentonites, silica, talcum, zinc oxide. For liquid dosage forms and emulsions suitable carriers are for example solvents, solubilizing agents, emulsifiers such as water, ethanol, isopropanol, ethyl carbonate, ethyl acetate, benzyl alcohol, benzyl benzoate, propylene glycol, 1,3-butyl glycol, cotton seed oil, peanut oil, olive oil, castor oil, sesame oil, glycerol fatty acid esters, polyethylglycols, fatty acid esters of sorbitan. Suspensions according to the disclosure may use carriers known in the art such as diluents (e.g. water, ethanol or propylene glycol), ethoxylated isostearyl alcohols, polyoxyethylene and polyoxyethylene sorbitan esters, microcrystalline cellulose, bentonites, agar agar, tragacanth.

The term binding agents refers to substances that bind powders or glue them together, rendering them cohesive through granule formation. They serve as a "glue" of the formulation. Binding agents increase the cohesive strength of the provided diluent or filler.

Suitable binding agents are for example starch from wheat, corn, rice or potato, gelatin, naturally occurring sugars such as glucose, sucrose or beta-lactose, sweeteners from corn, natural and synthetic gums such as acacia, tragacanth or ammonium calcium alginate, sodium alginate, carboxymethyl cellulose, sodium carboxymethyl cellulose, hydroxypropyl carboxymethyl cellulose, polyethylene glycol, polyvinyl pyrrolidone, magnesium aluminum silicate, waxes and others. The percentage of the binding agent in the composition can range from 1 - 30 % by weight, preferred 2 - 20 % by weight, more preferred 3 - 10 % by weight and most preferred 3 - 6 % by weight.

Colorants are excipients that bestow a colorization to the pharmaceutical formulation. These excipients can be food colorants. They can be adsorbed on a suitable adsorption means such as clay or aluminum oxide. A further advantage of a colorant is that it may visualize spilled aqueous solution on the nebulizer and/or the mouthpiece to facilitate cleaning. The amount of the colorant may vary between 0.01 and 10 % per weight of the pharmaceutical composition, preferred between 0.05 and 6 % per weight, more preferred between 0.1 and 4 % per weight, most preferred between 0.1 and 1 % per weight.

Suitable pharmaceutical colorants are for example curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulphite caramel, ammonia caramel, sulphite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminum, silver, gold, pigment rubine, tannin, orcein, ferrous gluconate, ferrous lactate.

Moreover, buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid), maleic acid, cacodylate (dimethyl arsenate), carbonic acid, ADA (N-(2-acetamido)imino diacetic acid, PIPES (4-piperazine-bis-ethanesulfonic acid), BIS-TRIS propane (1,3-bis[tris(hydroxymethyl)methylaminol] propane), ethylene diamine, ACES (2-[(amino-2-oxoethyl)amino]ethanesulfonic acid), imidazole, MOPS (3-(N-morphino) propanesulfonic acid), diethyl malonic acid, TES (2-[tris(hydroxymethyl)methyl]aminoethanesulfonic acid), HEPES (N-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazole, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), MOPS and N,N-bis-(2-hydroxyethyl)-2-aminoethanesulfonic acid (BES). Another group of preferred buffers are glycine, glycyl-glycine, glycyl-glycyl-glycine, N,N-bis-(2-hydroxyethyl)glycine and N-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, N,N,N-trimethyllysine, 3-methyl histidine, 5-hydroxy-lysine, o-phosphoserine, gamma-carboxyglutamate, [epsilon]-N-acetyl lysine, [omega]-N-methyl arginine, citrulline, ornithine and their derivatives.

Preservatives for liquid and/or solid dosage forms can be used on demand. They may be selected from the group comprising, but not limited to, sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorbutanol, thiomersal (sodium 2-(ethylmercurithio) benzoic acid), sulfur dioxide, sodium sulfite, sodium bisulfite, sodium metabisulfite, potassium metabisulfite, potassium sulfite, calcium sulfite, calcium hydrogen sulfite, potassium hydrogen sulfite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

The addition of a sufficient amount of antioxidants is particularly preferable for liquid and topical dosage forms. Suitable examples for antioxidants include sodium metabisulfite, alpha-tocopherol, ascorbic acid, maleic acid, sodium ascorbate, ascorbyl palmitate, butylated hydroxyanisol, butylated hydroxytoluene, fumaric acid or propyl gallate. Preferred is the use of sodium metabisulfite, alpha-tocopherol and ascorbyl palmitate.

Tablets or pills are usually coated, i.e., the coating constitutes the outer layer. This can be a film coating, a sugar coating with saccharides and a compression coating. Pharmaceutically acceptable varnishes or waxes, HPMC (hydroxypropylmethylcellulose), MC (methylcellulose) or HPC (hydroxypropylcellulose) can be used. Such a coating may help to disguise the taste, to ease the swallowing or the identification. Often plasticizers and pigments are included in the coating. Capsules normally have a gelatinous envelope that encloses the active substance. The specific composition and thickness of this gelatinous layer determines how fast absorption takes place after ingestion of the capsule. Of special interest are sustained release formulations, as known in the art.

Suitable sweeteners can be selected from the group comprising mannitol, glycerol, acesulfame potassium, aspartame, cyclamate, isomalt, isomaltitol, saccharin and its sodium, potassium and calcium salts, sucralose, alitame, thaumatin, glycyrrhizin, neohesperidine dihydrochalcone, steviol glycosides, neotame, aspartame-acesulfame salt, maltitol, maltitol syrup, lactitol, xylitol, erythritol.

Suitable thickening agents can be selected from the group comprising, but not limited to, polyvinyl pyrrolidone, methyl cellulose, hydroxypropyl methyl cellulose, hydroxypropyl cellulose, dextrins, polydextrose, modified starch, alkaline modified starch, bleached starch, oxidized starch, enzyme-treated starch, monostarch phosphate, distarch phosphate esterified with sodium trimetaphosphate or phosphorus oxychloride, phosphate distarch phosphate, acetylated distarch phosphate, starch acetate esterified with acetic anhydride, starch acetate esterified with vinyl acetate, acetylated distarch adipate, acetylated distarch glycerol, distarch glycerin, hydroxypropyl starch, hydroxy propyl distarch glycerin, hydroxypropyl distarch phosphate, hydroxypropyl distarch glycerol, starch sodium octenyl succinate, acetylated oxidized starch, hydroxyethyl cellulose.

Suitable pH-regulators for liquid dosage forms are e.g. sodium hydroxide, hydrochloric acid, buffer substances such as sodium dihydrogen phosphate or disodium hydrogen phosphate.

Suitable acidity regulators can be selected from the group comprising acetic acid, potassium acetate, sodium acetate, sodium diacetate, calcium acetate, carbon dioxide, malic acid, fumaric acid, sodium lactate, potassium lactate, calcium lactate, ammonium lactate, magnesium lactate, citric acid, mono-, di-, trisodium citrate, mono-, di-, tripotassium citrate, mono-, di-, tricalcium citrate, tartaric acid, mono-, disodium tartrate, mono-, dipotassium tartrate, sodium potassium tartrate, ortho-phosphoric acid, lecithin citrate, magnesium citrate, ammonium malate, sodium malate, sodium hydrogen malate, calcium malate, calcium hydrogen malate, adipic acid, sodium adipate, potassium adipate, ammonium adipate, succinic acid, sodium fumarate, potassium fumarate, calcium fumarate, ammonium fumarate, 1,4-heptonolactone, triammonium citrate, ammonium ferric citrate, calcium glycerophosphate, isopropyl citrate, potassium carbonate, potassium bicarbonate, ammonium carbonate, ammonium bicarbonate, magnesium carbonate, magnesium bicarbonate, ferrous carbonate, ammonium sulfate, aluminum potassium sulfate, aluminum ammonium sulfate, sodium hydroxide, potassium hydroxide, ammonium hydroxide, magnesium hydroxide, gluconic acid.

Acidifiers use to be inorganic chemicals that either produce or become acid. Suitable examples are: Ammonium chloride, calcium chloride.

Suitable solvents may be selected from the group comprising, but not limited to, water, carbonated water, water for injection, water with isotonizing agents, saline, isotonic saline, alcohols, particularly ethyl and n-butyl alcohol, and mixtures thereof.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

Penetration enhancers (permeation or permeability enhancers) are substances that temporarily diminish the barrier of the skin and promote or accelerate the absorption of cosmetic agents. Suitable penetration enhancers can be selected from the group comprising, but not limited to, dimethyl isosorbide (Arlasolve^{®}), dimethyl sulfoxide (DMSO) and its analogues, dimethyl formamide (DMF), azone (1-dodecylazacycloheptan-2-one), pyrrolidones such as 2-pyrrolidone, fatty acids such as oleic acid, lauric acid, myristic acid and capric acid, nonic surfactants such as polyoxyethylene-2-oleyl ether and polyoxyethylene-2-stearyl ether, terpenes, terpenoids and sesquiterpenes such as those from essential oils of eucalyptus, chenopodium and ylang-ylang, oxazolidinones such as 4-decyloxazolidin-2-one, turpentine oil, pine oil, menthol.

Suitable disintegrants can be selected from the group comprising starch, cold water-soluble starches such as carboxymethyl starch, cellulose derivatives such as methyl cellulose and sodium carboxymethyl cellulose, microcrystalline cellulose and cross-linked microcrystalline celluloses such as croscarmellose sodium, natural and synthetic gums such as guar, agar, karaya (Indian tragacanth), locust bean gum, tragacanth, clays such as bentonite, xanthan gum, alginates such as alginic acid and sodium alginate, foaming compositions a.o. Moisture expansion is supported by for example starch, cellulose derivatives, alginates, polysaccharides, dextrans, cross-linked polyvinyl pyrrolidone. The amount of the disintegrant in the composition may vary between 1 and 40% per weight, preferred between 3 and 20% per weight, most preferred between 5 and 10% per weight.

Glidants are materials that prevent a baking of the respective supplements and improve the flow characteristics of granulations so that the flow is smooth and constant. Suitable glidants comprise silicon dioxide, magnesium stearate, sodium stearate, starch and talcum. The amount of the glidant in the composition may vary between 0.01 and 10% per weight, preferred between 0.1 and 7% per weight, more preferred between 0.2 and 5% per weight, most preferred between 0.5 and 2% per weight.

The term "lubricants" refers to substances that are added to the dosage form in order to facilitate tablets, granulates etc. to be released from the press mold or the outlet nozzle. They diminish friction or abrasion. Lubricants are usually added shortly before pressing, as they should be present on the surface of the granules and between them and the parts of the press mold. The amount of the lubricant in the composition may vary between 0.05 and 15% per weight, preferred between 0.2 and 5% per weight, more preferred between 0.3 and 3% per weight, most preferred between 0.3 and 1.5% per weight. Suitable lubricants are a.o. sodium oleate, metal stearates such as sodium stearate, calcium stearate, potassium stearate and magnesium stearate, stearic acid, sodium benzoate, sodium acetate, sodium chloride, boric acid, waxes having a high melting point, polyethylene glycol.

Emulsifiers can be selected for example from the following anionic and non-ionic emulsifiers: Anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate, polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, propane-1,2-diol alginate, carrageenan, processed eucheuma seaweed, locust bean gum, tragacanth, acacia gum, karaya gum, gellan gum, gum ghatti, glucomannane, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, croscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate, calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristrearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch. Preferred are glycerin monooleate, stearic acid, phospholipids such as lecithin.

Suitable as surface-active solubilizing agents (solubilizers) are for example diethylene glycol monoethyl ester, polyethyl propylene glycol co-polymers, cyclodextrins such as α- and β-cyclodextrin, glyceryl monostearates such as Solutol HS 15 (Macrogol-15-hydroxystearate from BASF, PEG 660-15 hydroxystearates), sorbitan esters, polyoxyethylene glycol, polyoxyethylene sorbitanic acid esters, polyoxyethylene sorbitan monooleate, polyoxyethylene oxystearic acid triglyceride, polyvinyl alcohol, sodium dodecyl sulfate, (anionic) glyceryl monooleates.

Stabilizers are substances that can be added to prevent unwanted changes. Though stabilizers are not real emulsifiers they may also contribute to the stability of emulsions. Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Diluents or fillers are inactive substances added to drugs in order to handle minimal amounts of active agents. Examples for suitable diluents are water, mannitol, pre-gelatinized starch, starch, microcrystalline cellulose, powdered cellulose, silicified microcrystalline cellulose, dibasic calcium phosphate dihydrate, calcium phosphate, calcium carbonate, hydroxypropyl cellulose, hydroxyethyl cellulose, hydroxypropyl methylcellulose, polyethylene glycol, xanthum gum, gum arabic or any combination thereof.

Anti-caking agents (antiadherents) can be added to a supplement or a composition of supplements in order to prevent the formation of lumps and for easing packaging, transport, release from the at least one chamber of the dispensing cap and consumption. Suitable examples include tricalcium phosphate, powdered cellulose, magnesium stearate, sodium bicarbonate, sodium ferrocyanide, potassium ferrocyanide, calcium ferrocyanide, bone phosphate, sodium silicate, silicon dioxide, calcium silicate, magnesium trisilicate, talcum powder, sodium aluminosilicate, potassium aluminum silicate, calcium aluminosilicate, bentonite, aluminum silicate, stearic acid, polydimethyl siloxane.

Sorbents are materials that soak up oil from the water. Suitable examples include natural sorbents such as peat moss, sawdust, feathers, and anything else natural that contains carbon and synthetic sorbents such as polyethylene and nylon. Sorbents are used for tablet/capsule moisture-proofing by limited fluid sorbing (taking up of a liquid or a gas either by adsorption or by adsorption) in a dry state.

In some galenic formulations it may be desirable that a liquid oral dosage form generates some foam on being dissolved. Such an effect can be supported through the addition of a foaming agent that reduces the surface tension of the liquid, thus facilitating the formation of bubbles, or it increases its colloidal stability by inhibiting coalescence of bubbles. Alternatively, it may stabilize foam. Suitable examples include mineral oil, quillaia extract, triethyl citrate, sodium lauryl ether sulfate, sodium lauryl sulfate, ammonium lauryl sulfate.

Alternatively, some liquid oral dosage forms may appear slightly foamy upon preparation. Though this does not interfere with the desired application it may affect patient compliance in case of a medication or the commercial success in case of dietary supplements. Therefore, it may be desirable to add a pharmaceutically acceptable anti-foaming agent (defoamer). Examples are polydimethylsiloxane or silicone oil in dietary supplements or simethicone in pharmaceuticals.

Opacifiers are substances that render the liquid dosage for, opaque, if desired. They must have a refractive index substantially different from the solvent, in most cases here water. At the same time, they should be inert to the other components of the composition. Suitable examples include titanium dioxide, talc, calcium carbonate, behenic acid, cetyl alcohol, or mixtures thereof.

Suitable fatliquors are e.g. oleic acid decyl ester, hydrated castor oil, light mineral oil, mineral oil, polyethylene glycol, sodium laurylsulfate.

Consistency enhancers are e.g. cetyl alcohol, cetyl ester wax, hydrated castor oil, microcrystalline waxes, non-ionic emulsifier waxes, beeswax, paraffin or stearyl alcohol.

Suitable hydrotropes are alcohols such as ethanol, isopropyl alcohol or polyols such as glycerin.

Suitable aromatic and flavoring substances comprise above all essential oils that can be used for this purpose. In general, this term refers to volatile extracts from plants or parts of plants with the respective characteristic smell. They can be extracted from plants or parts of plants by steam distillation.

Suitable examples are: Essential oils, respectively aromatic substances from sage, cloves, chamomile, anise, star anise, thyme, tea tree, peppermint, mint oil, menthol, cineol, borneol, zingerol, eucalyptus oil, mango, figs, lavender oil, chamomile blossoms, pine needles, cypress, oranges, rosewood, plum, currant, cherry, birch leaves, cinnamon, limes, grapefruit, tangerine, juniper, valerian, lemon balm, lemon grass, palmarosa, cranberry, pomegranate, rosemary, ginger, pineapple, guava, echinacea, ivy leave extract, blueberry, kaki, melons etc. or mixtures thereof, as well as mixtures of menthol, peppermint and star anise oil or menthol and cherry flavor.

These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

According to the disclosure all of the aforementioned excipients and classes of excipients can be used without limitation alone or in any conceivable combination thereof, as long as the inventive use is not thwarted, toxic actions may occur, or respective national legislations are infracted.

The 6'-methoxycinchonan-9-ol according to the disclosure or one of its pharmaceutically acceptable salts and the further active ingredient can be used simultaneously, separately or sequentially in order to treat or prevent disease symptoms. The two active agents may be provided in a single dosage form or as separate formulation, each formulation containing at least one of the two active agents. One or both of the two active agents may be formulated as a bolus.

Pharmaceutical formulations suitable for oral dosage forms containing a 6'-methoxycinchonan-9-ol according to the disclosure or one of its pharmaceutically acceptable salts, a composition according to the disclosure or a combination according to the disclosure may be administered as separate units such as capsules, tablets, sugar-coated tablets or pills; powders or granulates; juices, syrups, drops, teas, solutions or suspensions in aqueous or non-aqueous liquids; edible foams or mousses; or in oil-in-water or water-in-oil in emulsions. In oral dosage forms such as a tablets or capsules the active agent can thus be combined with a non-toxic and pharmaceutically acceptable inert carrier such as ethanol, glycerol or water. Powders are produced by grinding the compound to a suitably tiny particle size and mixing them with a pharmaceutical carrier in a similar manner, e.g. an edible carbohydrate such as starch or mannitol. A flavor, preservative, dispersant or colorant can also be present. Tablets are formulated by producing, granulating or dry-pressing a powder mixture, adding a lubricant and a disintegrants and pressing the mixture to a tablet. A powder mixture is produced by mixing a suitably ground compound with a diluent or a base as described before, and if applicable, with a binding agent such as carboxymethyl cellulose, an alginate, gelatin or polyvinyl pyrrolidone, a dissolution retardant, such as, for example, paraffin, an absorption accelerator, such as, for example, a quaternary salt, and/or an absorbent, such as, for example, bentonite, kaolin or dicalcium phosphate. The powder mixture can be granulated by wetting it with a binder, such as, for example, syrup, starch paste, acacia mucilage or solutions of cellulose or polymer materials and pressing it through a sieve. As an alternative to granulation, the powder mixture can be run through a tableting machine, giving lumps of non-uniform shape which are broken up to form granules. The granules can be lubricated by addition of stearic acid, a stearate salt, talc or mineral oil in order to prevent sticking to the tablet casting mold. The lubricated mixture is then pressed to give tablets. The compounds according to the disclosure can also be combined with a free-flowing inert excipient and then pressed directly to give tablets without carrying out the granulation or dry-pressing steps.

In another aspect of the application a 6'-methoxycinchonan-9-ol according to the disclosure or one of its pharmaceutically acceptable salts, a composition according to the disclosure or a combination according to the disclosure is provided in hard gelatin capsules. They are fabricated by producing a powder mixture as described before and filling it into shaped gelatin covers. Glidants and lubricants such as highly dispersed silica, talcum, magnesium stearate, calcium stearate or polyethylene glycol can be added to the powder mixture as solids. A disintegrant or solubilizer such as agar agar, calcium carbonate or sodium carbonate can be added likewise in order to improve the availability of the medication after intake of the capsule. Additionally, suitable binding agents and/or colorants can be added to the mixture, if desirable or necessary.

In another aspect of the application a 6'-methoxycinchonan-9-ol according to the disclosure or one of its pharmaceutically acceptable salts, a composition according to the disclosure or a combination according to the disclosure is included in soft gelatin capsules (SGC). SGCs are dissolved on their passage through the gastrointestinal tract. They consist mainly of gelatin enriched with variable amounts of plasticizers such as glycerol or sorbitan. The release rate depends on the specific formulation of the SGC carrier material. They are also suitable for a sustained release of the active agent. SGCs are particularly useful for the administration of poorly water-soluble active agents.

Thus, in another aspect of the application the present application relates to a 6'-methoxycinchonan-9-ol according to the disclosure for use in the prophylaxis or treatment of a coronaviral infection in a formulation for oral administration, as defined in the claims.

In yet another aspect of the application the present application relates to a 6'-methoxycinchonan-9-ol according to the disclosure for use in the prophylaxis or treatment of a coronaviral infection in a formulation for inhalatory administration (not part of the claimed invention).

For an effective prophylactic or therapeutic treatment of coronavirus infections that may cause pneumonia, pulmonary edema and/or acute lung injury with at least one 6'-methoxycinchonan-9-ol according to the disclosure it is advantageous to reach the patient's alveoli. Therefore, the particle size must be sufficiently small to reach the lowest parts of the airways of the pulmonary tissue. The best inhalatory device class for inhalatory application of a pharmaceutically active agent are the so-called mesh nebulizers described before. In the scope of the present application practically all mesh nebulizers known in the art can be used, from rather simple single-use mesh nebulizers for cough and cold or for fancy purposes to sophisticated high-end mesh nebulizers for clinical or domestic treatment of serious diseases or conditions of the lower airways.

Suitable commercially available mesh nebulizers, jet nebulizers, ultrasonic nebulizers, dry powder inhalers and (pressurized) metered-dose inhalers comprise, without being limiting, eFlow^{®}rapid, PARI LC STAR^{®}, PARI Velox and PARI Velox Junior (PARI GmbH, Starnberg, Germany), Philips Respironics I-neb and Philips InnoSpire Go (Koninklijke Philips N.V., Eindhoven, Netherlands), VENTA-NEB^{®}-ir, OPTI-NEB^{®}, M-neb^{®} dose⁺ mesh nebulizer inhalation MN-300/8, M-Neb Flow+ and M-neb^{®} mesh nebulizer MN-300/X (NEBU-TEC, Eisenfeld, Germany), Homed Deepro HCM-86C and HCM860 (HCmed Innovations Co., Ltd, Taipei, Taiwan), OMRON MicroAir U22 and U100 (OMRON, Kyoto, Japan), Aerogen^{®} Solo, Aerogen^{®} Ultra and Aerogen^{®} PRO (Aerogen, Galway, Ireland), KTMED NePlus NE-SM1 (KTMED Inc., Seoul, South Korea), Vectura Bayer Breelib^{™} (Bayer AG, Leverkusen, Germany), Vectura Fox, MPV Truma and MicroDrop^{®} Smarty (MPV MEDICAL GmbH, Kirchheim, Germany), MOBI MESH (APEX Medical, New Taipei City, Taiwan), B.Well WN-114, TH-134 and TH-135 (B.Well Swiss AG, Widnau, Switzerland), Babybelle Asia BBU01 (Babybelle Asia Ltd., Hongkong), CA-MI Kiwi and others (CA-MI sri, Langhirano, Italy), Diagnosis PRO MESH (Diagnosis S.A., Bia 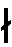 ystok, Poland), DIGI O₂ (DigiO₂ International Co., Ltd., New Taipei City, Taiwan), feellife AIR PLUS, AEROCENTRE+, AIR 360+, AIR GARDEN, AIRICU, AIR MASK, AIRGEL BOY, AIR ANGEL, AIRGEL GIRL and AIR PRO 4 (Feellife Health Inc., Shenzhen, China), Hannox MA-02 (Hannox International Corp., Taipei, Taiwan), Health and Life HL100 and HL100A (HEALTH & LIFE Co., Ltd., New Taipei City, Taiwan), Honsun NB-810B (Honsun Co., Ltd., Nantong, China), K-jump^{®} KN-9100 (K-jump Health Co., Ltd., New Taipei City, Taiwan), microlife NEB-800 (Microlife AG, Widnau, Switzerland), OK Biotech Docspray (OK Biotech Co., Ltd., Hsinchu City, Taiwan), Prodigy Mini-Mist^{®} (Prodigy Diabetes Care, LLC, Charlotte, USA), Quatek NM211, NE203, NE320 and NE403 (Big Eagle Holding Ltd., Taipei, Taiwan), Simzo NBM-1 and NBM-2 (Simzo Electronic Technology Ltd., Dongguan, China), Mexus^{®} BBU01 and BBU02 (Tai Yu International Manufactory Ltd., Dongguan, China), TaiDoc TD-7001 (TaiDoc Technology Co., New Taipei City, Taiwan), Vibralung^{®} and HIFLO Miniheart Circulaire II (Westmed Medical Group, Purchase, USA), KEJIAN (Xuzhou Kejian Hi-Tech Co., Ltd., Xuzhou, China), YM-252, P&S-T45 and P&S-360 (TEKCELEO, Valbonne, France), Maxwell YS-31 (Maxwell India, Jaipur, India), Kernmed^{®} JLN-MB001 (Kernmed, Durmersheim, Germany).

Preferred are mesh nebulizers with a piezoelectric activation of the nebulization process, respectively vibrating mesh nebulizers.

Thus, in another aspect of the application the present application relates to a 6'-methoxycinchonan-9-ol according to the disclosure for use in a formulation in the prophylaxis or treatment of a coronaviral infection for inhalatory administration (not part of the claimed invention), wherein the inhalatory administration is carried out by means of a vibrating mesh nebulizer.

Mesh nebulizers can be classified into two groups according to patient interaction: Continuous mode devices and trigger-activated devices. In continuous mode mesh nebulizers the nebulized aerosol is continuously released into the mouth piece and the patient has to inhale the provided aerosol. In trigger-activated devices a defined amount of aerosol is released only upon an active and deep inspiratory breath. This way a far larger amount of active agent-containing aerosol is inhaled and reaches the lowest airways than with continuous mode devices. The latter lose a large amount of active agent-containing aerosol either to the surrounding or on the passage of the upper airways, as the aerosol release is not coupled to the respiratory cycle.

Therefore, trigger-activated mesh nebulizers are preferred, in particular vibrating mesh nebulizers.

Particularly preferred are trigger-activated mesh nebulizers with a piezoelectric activation of the nebulization process.

Preferred are the mesh nebulizer models PARI eFlow^{®}rapid, Philips Respironics I-neb, Philips InnoSpire Go, M-neb^{®} dose⁺ mesh nebulizer inhalation MN-300/8, Homed Deepro HCM-86C and HCM860, OMRON MicroAir U100, Aerogen^{®} Solo, KTMED NePlus NE-SM1, Vectura Fox, Vectura Bayer Breelib^{™}.

The most preferred vibrating mesh nebulizer models are high-end models such as PARI eFlow^{®}rapid, PARI Velox, Philips Respironics I-neb, M-neb^{®} dose⁺ mesh nebulizer inhalation MN-300/8, Aerogen^{®} Solo, Vectura Fox, Vectura Bayer Breelib^{™}.

The mean droplet size is usually characterized as MMAD (median mass aerodynamic diameter). The individual droplet size is referred to as MAD (mass aerodynamic diameter). This value indicates the diameter of the nebulized particles (droplets) at which 50% are smaller or larger, respectively. Particles with a MMAD > 10 µm normally do not reach the lower airways, they often get stuck in the throat. Particles with a MMAD > 5 µm and < 10 µm usually reach the bronchi but not the alveoli. Particles between 100 nm and 1 µm MMAD don't deposit in the alveoli and are exhaled immediately. Therefore, the optimal range is between 1 µm and 5 µm MMAD. Recent publications even favor a narrower range between 3.0 µm and 4.0 µm (cf. Amirav et al. (2010) J Allergy Clin Immunol 25: 1206-1211; Haidl et al. (2012) Pneumologie 66: 356-360).

A further commonly accepted quality parameter is the percentage of the particles in the generated aerosol with a diameter in the range of 1 µm to 5 µm (FPM; fine particle mass). FPM is a measure for the particle distribution. It is calculated by subtracting the percentage of the particles in the generated aerosol with a diameter in the range < 1 µm from the overall percentage of the particles in the generated aerosol with a diameter in the range < 5 µm (FPF; fine particle fraction).

In another non-claimed aspect the present application refers also to a method for producing an aerosol according to the disclosure, comprising the following steps:
a) filling 0.1 ml to 5 ml of an aqueous solution containing the at least one 6'-methoxycinchonan-9-ol according to the disclosure and optionally at least one pharmaceutically acceptable excipient into the nebulization chamber of a mesh nebulizer,
b) starting vibration of the mesh of the mesh nebulizer at a frequency of 80 kHz to 200 kHz, and
c) discharging the generated aerosol at the side of the mesh of the mesh nebulizer opposite to the nebulization chamber.

The vibration frequency of vibrating mesh nebulizers is normally in the range of 80 kHz to 200 kHz, preferred 90 kHz to 180 kHz, more preferred 100 kHz to 160 kHz, most preferred 105 kHz to 130 kHz (cf. Chen, The Aerosol Society: DDL2019**;** Gardenshire et al. (2017) A Guide to Aerosol Delivery Devices for Respiratory Therapists, 4th ed.).

Thus, the aforementioned method is also disclosed with said vibration frequency ranges. The method according to the disclosure is thus characterized in that at least 80 % in weight, preferred at least 85 % in weight, most preferred at least 90 % in weight of the at least one 6'-methoxycinchonan-9-ol according to the disclosure contained in said aqueous solution are nebulized in the generated aerosol.

The method of the disclosure is particularly effective in nebulizing a high percentage of the pharmaceutically active agent(s) from the provided aqueous solution during a short time. This is an important feature for patient compliance. A considerable percentage of the patient population finds the inhalatory process to be uncomfortable, weary and physically demanding. On the other hand, the patient's active cooperation is essential for an effective and targeted inhalatory application. Therefore, it is desirable that a therapeutically sufficient amount is applied during a period of time as short as possible. During a three minutes time span 95 % of the substance provided in the aqueous solution can be nebulized. This is an ideal time span for a high patient compliance.

Therefore, the method according to the disclosure is thus characterized in that at least 80 % of the generated aerosol are produced during three minutes after starting nebulization in the mesh nebulizer, preferred at least 85 % and most preferred at least 90 %.

While the pharmaceutically active agent is usually provided in a single dosage container for every nebulization procedure the nebulizer and/or the mouthpiece can be used over a certain period of time and have to be replaced at certain intervals. A cleaning of the nebulizer and the mouthpiece is recommended by default after each nebulization. But herein patient compliance cannot be reasonably taken for granted. But even after a meticulous cleaning there are always some deposits of the aerosol in the nebulization chamber, the outlet and/or the mouthpiece. As the aerosol is produced from an aqueous solution these depositions bear the risk of producing a bioburden of bacteria that might contaminate the inhaled aerosol. Deposits may also plug holes in the mesh membrane of the mesh nebulizer. In general, the nebulizer and/or the mouthpiece should be exchanged every one or two weeks. Therefore, it is convenient to offer the medication and the nebulizer as a combined product.

Thus, in another non-claimed aspect the present application refers also to a kit comprising a mesh nebulizer and a pharmaceutically acceptable container with an aqueous solution containing the at least one 6'-methoxycinchonan-9-ol according to the disclosure and optionally at least one pharmaceutically acceptable excipient.

In an alternative kit the at least one 6'-methoxycinchonan-9-ol according to the disclosure is not provided in form of an aqueous solution but in two separated containers, one for a solid form for the active agent and the other for an aqueous solution. The final aqueous solution is freshly prepared by solving the active agent in the final solution. Thereupon the final aqueous solution is filled into the nebulization chamber of the mesh nebulizer. These two containers can be completely separated containers, e.g. two vials, or e.g. a dual-chamber vial. For solving the active agent e.g. a membrane between the two chambers is perforated to allow for mixing of the content of both chambers.

Thus, the present application discloses also a kit, comprising a mesh nebulizer, a first pharmaceutically acceptable container with water for injection or physiological saline solution and a second pharmaceutically acceptable container with a solid form of the at least one a 6'-methoxycinchonan-9-ol according to the disclosure, wherein optionally at least one pharmaceutically acceptable excipient is contained in the first pharmaceutically acceptable container and/or the second pharmaceutically acceptable container.

The aerosol generated by the method according to the disclosure is administered, respectively self-administered by means of a mouthpiece. Optionally, such a mouthpiece can be additionally included in the beforementioned kits.

A common way to transfer the provided aqueous solution or final aqueous solution into the nebulization chamber of the mesh nebulizer by means of a syringe equipped with an injection needle. First, the aqueous solution is drawn up into the syringe and then injected into the nebulization chamber. Optionally, such a syringe and/or injection needle can be additionally included in the beforementioned kits. Without being limiting, typical syringes made of polyethylene, polypropylene or cyclic olefin co-polymers can be used, and a typical gauge for a stainless steel injection needle would be in the range of 14 to 27.

In yet another non-claimed aspect the present application discloses a 6'-methoxycinchonan-9-ol according to the disclosure or one of its pharmaceutically acceptable salts, a composition according to the disclosure or a combination according to the disclosure for use in the prophylaxis or treatment of acute lung injury, wherein said substance, composition or combination is provided as an additive to the ventilation air of a cardiopulmonary bypass device, a form of assisted ventilation. When the patient's condition in intensive care unit worsens, they often need to be ventilated in such a device for an indefinite period of time until their own respiration would allow for a sufficient oxygen supply. Good results have been achieved when with an aerosol in a metered-dose inhaler combined with an inhalation chamber at the Y-piece. This can increase the applied dosage of bronchodilators by the factor 1.5 to 4 (Fuller et al. (1994) Chest 105: 214-218). 38% of the pharmaceutically active agent could be delivered (Marik et al. (1999) Chest 115: 1653-1657). Alternatively, a constant output mesh nebulizer yielded rates of 10 - 15%, as assessed in a scintigraphic study (Dugernier et al. (2016) Ann Intensive Care 6: 73). Vibrating mesh nebulizers delivered better results than ultrasonic or jet nebulizers for administration of antibiotics. When a constant output vibrating-mesh nebulizer is placed on the inspiratory limb at 10 cm of the Y-piece and specific ventilation parameters (tidal volume of 8 ml/kg, respiratory rate of 12 c/min, duty cycle of 50%, constant and low inspiratory flow rate inferior to 30 l/min and end inspiratory pause of 20%) are set, 63% of the administered drug (ceftazidime, amikacin) reach the inlet of the endotracheal tube, versus 37% extrapulmonary deposition (Lu et al. (2011) Am J Respir Crit Care Med 184: 106-115). Mostly, the administered drug is evenly distributed between both lungs. In pigs, the use of helium (He/O₂) instead of nitrogen (N₂/O₂) in inhaled gas was found to increase ceftazidime concentrations in subpleural lung specimens (Tonnelier et al. (2005) Anesthesiology 102: 995-1000).

In these cases, a 6'-methoxycinchonan-9-ol according to the disclosure can be added to the intubated ventilation air in solid form (dry powder) or in liquid form (in an aqueous solution or as a nebulized aerosol, as described before).

The present application discloses thus also 6'-methoxycinchonan-9-ol or one of its pharmaceutically acceptable salts, a composition according to the disclosure or a combination according to the disclosure for use in the prophylaxis or treatment of acute lung injury, wherein said substance, composition or combination is added to the ventilation air of a cardiopulmonary bypass device. device (not part of the claimed invention).

In yet another aspect the present application relates to a 6'-methoxycinchonan-9-ol according to the disclosure for use in the prophylaxis or treatment of a coronaviral infection in a formulation for sublingual tablets, as defined in the claims.

In yet another aspect the present application relates to a 6'-methoxycinchonan-9-ol according to the disclosure for use in the prophylaxis or treatment of a coronaviral infection in a formulation as a liquid dosage form.

In general, an aqueous solution or a physiological saline solution is preferred. In case of a poorly soluble pharmaceutical agent according to the disclosure also ethanol or ethanol/water mixtures can be used.

Suitable liquid dosage forms include drops, eyedrops, eardrops or injection solutions.

The present application discloses also the parenteral administration of a 6'-methoxycinchonan-9-ol according to the disclosure in the prophylaxis or treatment of a coronaviral infection in the form of intravenous injection, intraarterial injection or intraperitoneal injection (not part of the claimed invention).

These liquid dosage forms comprise solutions, suspensions and emulsions. Examples are water and water/propylene glycol solutions for parenteral injections, or the addition of a sweetener or opacifier for oral solutions, suspensions and emulsions.

These liquid dosage forms can be stored in vials, IV bags, ampoules, cartridges, and prefilled syringes. Suitable excipients include solubilizers, stabilizers, buffers, tonicity modifiers, bulking agents, viscosity enhancers/reducers, surfactants, chelating agents, and adjuvants. While SARS-CoV and MERS-CoV infect above all the lower airways SARS-CoV-2 infects first the pharynx/throat area. Only a minor percentage of these patients develops later a pulmonary infection and a pneumonia. While these pharyngeal infections cause usually only mild symptoms as in a cold or no symptoms at all these patients are highly infectious for their environment. In most cases they are unaware that they have become spreaders of the infection. Therefore, there is a medical need to treat coronaviral infections already when they are still in the pharyngeal stage, not only for treating such a patient but also for epidemiologic reasons to prevent the spreading of the epidemic. For patients with a pharyngeal infection only a systemic route of administration, e.g. intravenously or perorally, with a highly effective drug or drug combination that may also cause adverse side effects is not ideal. Thus, it is desirable to provide routes of administration that treat the infected pharyngeal tissue locally.

Therefore, in yet another aspect the present application relates a 6'-methoxycinchonan-9-ol according to the disclosure for use in the prophylaxis or treatment of a coronaviral infection in a formulation for pharyngeal administration, as defined in the claims.

Administration of a medication to the pharynx can be effected by topical administrations, such as brushing of the throat/pharynx area with a suitable liquid dosage form as drops, a lotion or a tincture, or with a viscous dosage form such as a gel or hydrogel, gurgling with a mouthwash, a sublingual tablet, a lozenge, a throat spray or a posterior pharyngeal wall injection.

A lotion is a low-viscosity topical preparation intended for application to the skin or the mucosa. Lotions are applied to the skin or mucosa with bare hands, a brush, a clean cloth, or cotton wool.

An advantage of a lotion is that it may be spread thinly and may cover a large area of skin or mucosa. Typical drugs that can be administered in form of a lotion include antibiotics, antiseptics, antifungals, corticosteroids, anti-acne agents, soothing, smoothing, moisturizing or protective agents, or anti-allergens.

Most lotions are oil-in-water emulsions using a substance such as cetearyl alcohol to keep the emulsion together, but water-in-oil lotions are also formulated. The key components are the aqueous and oily phases, an emulgent to prevent separation of these two phases and the drug substance(s). A wide variety of excipients such as fragrances, glycerol, petroleum jelly, dyes, preservatives, proteins and stabilizing agents are commonly added to lotions. Thickness, consistency and viscosity of the lotion can be adjusted during manufacturing. Manufacturing lotions can be carried out in two cycles: a) Emollients and lubricants are dispersed in oil with blending and thickening agents. b) Perfume, color and preservatives are dispersed in the water phase. Pharmaceutically active principles are broken up in both cycles depending on the raw materials involved and the desired properties of the lotion.

A tincture is typically an alcoholic extract or formulation. Solvent concentrations of 25-60% (or even 90%) are common. Other solvents for producing tinctures include vinegar, glycerin, diethyl ether and propylene glycol. Ethanol has the advantage of being an excellent solvent for both acidic and alkaline constituents. A tincture using glycerin is called a glycerite. Glycerin is generally a poorer solvent than ethanol. Vinegar, being acidic, is a better solvent for obtaining alkaloids but a poorer solvent for acidic components.

A gel is a colloid in which the solid disperse phase forms a network in combination with that of the fluid continuous phase, resulting in a viscous semirigid sol. Gel properties range from soft and weak to hard and tough. They are defined as a substantially dilute cross-linked system, which exhibits no flow in the steady-state. By weight, gels are mostly liquid, yet they behave like solids due to a three-dimensional cross-linked network within the liquid. It is the crosslinking within the fluid that gives a gel its consistency and contributes to the adhesive stick. Gels are a dispersion of molecules of a liquid within a solid medium.

A hydrogel is a network of polymer chains that are hydrophilic, sometimes found as a colloidal gel in which water is the dispersion medium. A three-dimensional solid results from the hydrophilic polymer chains being held together by cross-links. Because of the inherent cross-links, the structural integrity of the hydrogel network does not dissolve from the high concentration of water. Hydrogels are highly absorbent (they can contain over 90% water) natural or synthetic polymeric networks. Hydrogels also possess a degree of flexibility very similar to natural tissue, due to their significant water content. In medicine, hydrogels can encapsulate chemical systems which upon stimulation by external factors such as a change of pH may cause specific pharmaceutically active agent(s) to be liberated to the environment, in most cases by a gel-sol transition to the liquid state.

Suitable gel formers can be selected from the group comprising, but not limited to, agar, algin, alginic acid, bentonite, carbomer, carrageenan, hectorite, hydroxyethyl cellulose, hydroxypropyl cellulose, polyvinyl alcohol, polyvinyl pyrrolidone, sodium carbomer.

A mouthwash is a liquid which is held in the mouth passively or swilled around the mouth by contraction of the perioral muscles and/or movement of the head, and may be gargled, where the head is tilted back and the liquid bubbled at the back of the mouth. An aqueous or alcoholic solution of a 6'-methoxycinchonan-9-ol according to the disclosure can thus be formulated and administered to the pharynx.

Sublingual drug delivery can be an alternative when compared to oral drug delivery as sublingually administered dosage forms bypass hepatic metabolism. A rapid onset of pharmacological effect is often desired for some drugs, especially those used in the treatment of acute disorders. Sublingual tablets disintegrate rapidly, and the small amount of saliva present is usually sufficient for achieving disintegration of the dosage form coupled with better dissolution and increased bioavailability.

The drug must be lipophilic enough to be able to partition through the lipid bilayer, but not so lipophilic such that once it is in the lipid bilayer, it will not partition out again. According to the diffusive model of absorption, the flux across the lipid bilayer is directly proportional to the concentration gradient. Therefore, lower salivary solubility results in lower absorption rates and vice versa. In general, a drug which has been formulated for sublingual should ideally have a molecular weight of less than 500 to facilitate its diffusion. The oral cavity has a narrow pH range which lies between 5.0 to 7.0. The inclusion of a suitable buffer during the formulation of an ionizable drug makes it possible to control the pH of aqueous saliva.

In order to avoid a possibly unpleasant taste or smell of the drug taste masking is needed. Sweeteners, flavors, and other taste-masking agents are essential components. Sugar-based excipients quickly dissolve in saliva and produce endothermic heat of dissolution. They create a pleasant feeling in the mouth and are most suitable for sublingual tablets along with other flavors.

Typical techniques for manufacturing sublingual tablets include direct compression, compression molding, freeze drying and hot melt extrusion (Khan et al. (2017) J Pharmaceut Res 16: 257-267).

When swallowing is avoided, an administration of a pharmaceutically active agent by means of a sublingual tablet can also reach the pharynx/throat topically. Absorption of the pharmaceutically active agent occurs to a good part via the pharyngeal mucosa.

A lozenge (troche) is a small, disc-shaped or rhombic body composed of solidifying paste containing an astringent, antiseptic, or demulcent drug, used for local treatment of the mouth or throat, the lozenge being held in the mouth until dissolved. The vehicle or base of the lozenge is usually sugar, made adhesive by admixture with acacia or tragacanth, fruit paste, made from black or red currants, confection of rose, or balsam of tolu.

In particular, the present application relates to a 6'-methoxycinchonan-9-ol according to the disclosure for use in the prophylaxis or treatment of a coronaviral infection in a formulation for pharyngeal administration, wherein the pharyngeal administration is carried out by means of a throat spray.

A throat spray is a medicated liquid administered to the throat as a spray, typically for the treatment of a sore throat or cough.

A throat spray may typically contain a local anesthetic (e.g. lidocaine, benzocaine), an antiseptic (e.g. chlorhexidine, cetylpyridinium chloride), herbal extracts or a combination thereof. Whatever the formulation, it should not contain too much sugar or ethanol, which further irritates the mucosa. And finally, the user should not experience any unpleasant aftertaste.

The standard for throat sprays is currently a metering pump attached to a bottle containing between 10 to 30 ml of a liquid formulation. The formulation is filled into a glass or plastic bottle with the pump fixed by a screw closure, crimped on or simply snapped onto the bottle neck. Irrespective of the fixing option selected, the system should be tight, with no leakage observed during carrying or handling by the user. Usually, the container is made from glass or plastic.

Typically, a throat spray pump will deliver a dose in the range of 50 to 200 µl per actuation. For a targeted administration, the pump will be equipped with an actuator with a prolonged nozzle. The nozzle length may range from 30 to 70 mm. It is easier to target the affected area with such a long-fixed nozzle, but this can be too bulky for users to carry, which is why actuators with foldable or swivel-mounted nozzles are preferred.

Alternatively, devices utilize continuous valves. A continuous valve delivers a targeted treatment but not precise dosing, as the formulation will be aerosolized while the actuator is pressed down. One technical solution is a tin or aluminum can with pressurized head space. When actuating the valve, the elevated internal pressure will force the formulation out of the can as long as the valve stem is pressed down.

A related but more sophisticated system is the bag-on-valve (BOV) system. The product is placed inside a bag while a propellant (in most cases compressed air) is filled in the space between the bag and the outer can. The product is squeezed out of the bag by the compressed air when the continuous valve is actuated. A BOV system will work with any 360° orientation.

Care should be taken, as throat spray formulations may contain ingredients that are very aggressive and can lower the surface tension. A simple test for spray performance will ensure that the formulation can be aerosolized by the system and that the delivered spray pattern and particle size is appropriate for the intended use.

Spray pattern and droplet size distribution are the most important parameters for a throat spray. Spray pattern is a term used to describe the spray angle and the shape of the plume for a fully developed spray. The droplet size is characterized once the spray is fully developed using a laser diffraction method. Fine particles (droplets with less than 10 µm mean dynamic diameter) should be as low as possible to avoid droplet deposition in the lower airways.

Recently, some carragelose-based throat sprays emerged, claiming protection to virus born upper respiratory infections. The first polymer of this platform is Carragelose^{®}, a broadly active anti-viral compound for treating respiratory diseases. The compound prevents the binding of viruses on the mucosal cells, in addition to its moistening effect.

Alternatively, a portable nebulizer with a high output rate and a tuned droplet size for deposition in the upper airways can be used. Breathing through a face mask can deposit droplets on the mucosa of the whole upper airways (cf. Marx and Nadler (2018) Drug Development & Delivery).

In particular, the present application relates a 6'-methoxycinchonan-9-ol according to the disclosure for use in the prophylaxis or treatment of a coronaviral infection in a formulation for pharyngeal administration, wherein the pharyngeal administration is carried out by means of a posterior pharyngeal wall injection.

This technique is used for pharyngoplasty by injection of calcium hydroxylapatite and other methods in plastic surgery. However, also a local injection can be made into the pharyngeal tissue in order to administer a pharmaceutically active agent. The injection solution can be roughly the same as for intravenous or intramuscular injections. Preferred are aqueous solutions, physiological saline solutions or, in case of a rather lipophilic pharmaceutically active agent, an ethanol/water mixture.

It is known that the eye mucosae are another entry point of SARS-CoV-2 to the organism, e.g. a person carries the viruses on his hands while rubbing his eyes.

Therefore, the present application relates also to a 6'-methoxycinchonan-9-ol according to the disclosure, wherein the 6'-methoxycinchonan-9-ol according to the disclosure is provided in a formulation of eye drops (not part of the claimed invention).

Eye drops are mostly aqueous solutions containing a pharmaceutically active agent. The pH is usually adjusted to 7.1 to 7.5. Common buffers for eye drops are boric acid and monobasic sodium phosphate. The tonicity should be adjusted by 0.9 % saline (or another isotonizing agent such as potassium nitrate, boric acid, sodium acetate, sodium acetate phosphate buffer or mannitol) to an osmotic pressure isotonic to the cornea epithelium (225 - 430 mosm/kg). Suitable preservatives include thiomersal, organic mercury compounds such as phenylmercury, benzalkonium chloride, chlorhexidine and benzylic alcohol. For prolonging the contact time viscosity-increasing substances (thickening agents) such as cellulose derivatives (hypromellose, methylcellulose, hydroxypropyl methylcellulose), hyaluronic acid, cellulose acetate phthalate, polyethylene glycol, polyvinyl alcohols or poloxamers can be added. Wetting agents or surfactants such as benzalkonium chloride, polysorbate 20, polysorbate 80, dioctyl sodium sulphosuccinate can be included. Some amino acids, alone or in combination with sodium hyaluronate may be helpful in promoting tissue reconstitution, if needed. Suitable amino acids are glycine, leucine, lysine and proline (cf. EP 1940381 B1). Thus, the present disclosure refers also to a pharmaceutical composition for use as described before, wherein the pharmaceutical composition is a formulation for a throat spray, nose spray or eye drops. Only the formulation in a throat spray is part of the claimed invention.

In a further non-claimed aspect a method of treatment for a coronaviral infection is disclosed, in which an effective dose a 6'-methoxycinchonan-9-ol according to the disclosure is administered to a patient in need thereof.

Tautomerism relates to a rapid intraconversion of organic compounds in which a hydrogen atom or proton formally migrates inside the compound. This is accompanied by a switch of a single bond and adjacent double bond. The single forms are called tautomers. Thus, the present patent application refers also to the use of all tautomers of the at least one 6'-methoxycinchonan-9-ol according to the disclosure.

For some applications it may be desirable that isotopically enriched forms of the compounds of the disclosure are used, e.g., for diagnostic purposes. Thus, the present patent application refers also to such isotopically enriched forms of the compounds of the disclosure.

From a pharmacokinetic point of view or for a production rationale it may be preferable to use a prodrug as a dosage form. A prodrug is administered in a pharmacologically inactive form and is metabolically converted into the active form inside the body. This conversion may occur systemically or locally. Thus, the present patent application refers also to prodrugs of the compounds of the disclosure (not part of the claimed invention).

As used throughout the present application the terms "the at least one 6'-methoxycinchonan-9-ol shall encompass all the aforementioned molecular variants, unless otherwise stated.

### EXAMPLES

### Example 1: Quinine sulfate inhibits the replication of SARS-CoV-2 in infected Vero-B4 cells in a dose-dependent manner in Western Blots

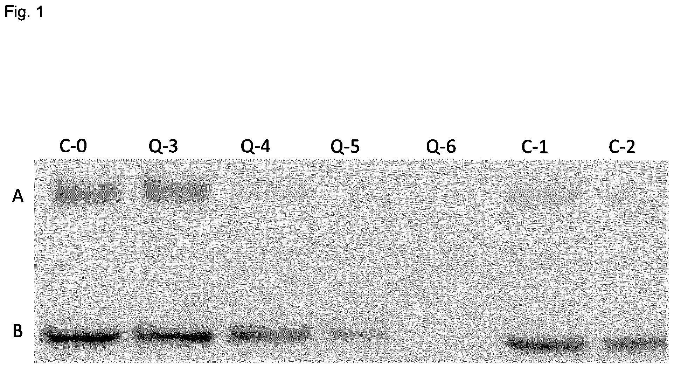

In order to investigate whether quinine has an effect on the spread of viral infection, Western Blot (WB) analyses were carried out. Vero-B4 cells (Meyer et al. (2015) Emerg Infect Dis 21: 181-182) were infected with SARS-CoV-2 for two hours. Cells were then washed with PBS (phosphate buffer saline), provided with fresh medium containing quinine sulfate in several non-cytotoxic concentration range (100 nM, 1 µM), 10 µM) and 100 µM). The treatment with quinine was carried out over the entire experimental procedure. 3 days post infection (dpi) cells and the virus-containing supernatants were harvested. Then, virions were purified from the cell culture supernatants via a 20% sucrose cushion. The virions were denaturized in SDS (sodium dodecyl sulfate) sample buffer, separated by SDS gel electrophoresis and transferred to a nitrocellulose membrane. SARS-CoV-2 were visualized using a convalescent serum and a horseradish peroxidase-coupled secondary reagent by means of an electrochemiluminescence reaction. Herein, a dose-dependent inhibition of SARS-Cov-2 replication was shown. At a concentration of 100 µM) quinine sulfate showed a complete block of virus replication (Fig.1). The reduction was significantly higher than after treatment with 10 µM) chloroquine or 10 µM) hydroxychloroquine (compounds known to reduce SARS-CoV-2 load) which were used as positive control.

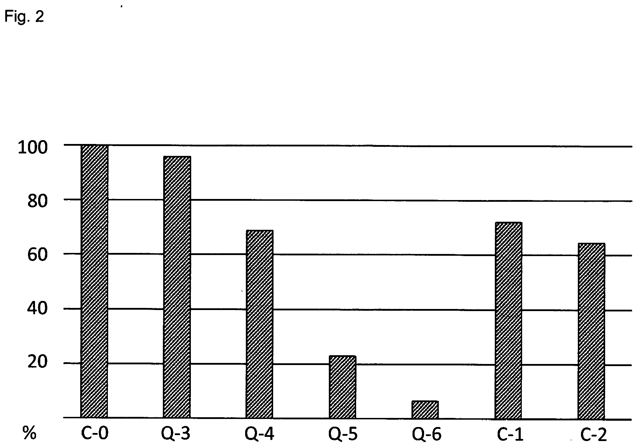

Densitometric evaluations of SARS-CoV-2 Nucleoprotein in the virus fraction were carried out with the analysis program AIDA^{®}. The densitometric evaluation allows for the quantification of signal intensities in Western Blot and thus for conclusions on the quantity of a certain protein in the sample. The evaluation showed clearly that after the addition of quinine sulfate the generation of SARS-CoV-2 proteins is inhibited in a dose-dependent manner, even to a higher percentage as for the same concentration of chloroquine or hydroxychloroquine (Fig. 2).

### Example 2: Quinine sulfate inhibits the replication of SARS-CoV-2 in infected Vero-B4 cells in a dose-dependent manner in qRT-PCR

To evaluate these results qRT-PCR (quantitative real-time polymerase chain reaction) experiments were conducted, a very sensitive method to determine quantitatively the number of SARS-CoV-2 RNA copies in infective virions released from cells in the supernatant. Vero-B4 cells were infected with SARS-CoV2 for 1 h. Cells were then washed with PBS, provided with fresh medium containing quinine sulfate in a non-cytotoxic concentration range (100 nM, 1 µM), 10 µM) and 100 µM). The treatment with quinine sulfate was carried out over the entire experimental procedure. 3 days post infection (dpi) the virus-containing supernatants were harvested. Samples containing the released virions were quantified by real-time PCR AgPath-ID One-Step RT-PCR Kit from Ambion (Cat: 4387424), allowing reverse transcription, cDNA synthesis and PCR amplification in a single step. Samples were analyzed by 7500 software v2.3 (applied Bioscience).

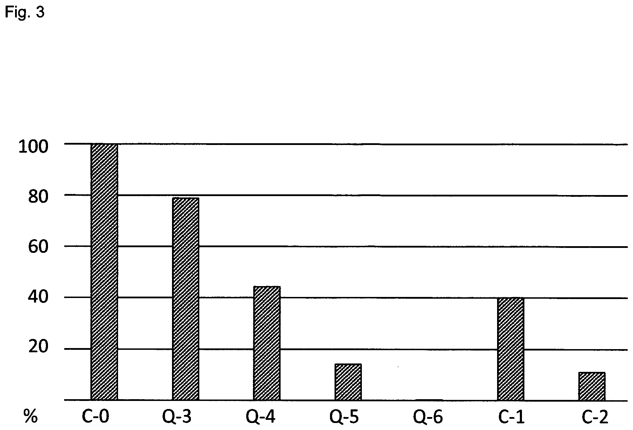

Using this very sensitive method a dose-dependent reduction in the amount of SARS-Cov-2 RNA copies could be shown (Fig.3). At a concentration of 10 µM) quinine sulfate a reduction of approximately 85% and at 100 µM) a complete block of virus spread was detected. This confirms the results from Example 1. The reduction was again significantly higher than after treatment with 10 µM) chloroquine or 10 µM) hydroxychloroquine.

### Example 3: In effective doses quinine sulfate is not cytotoxic in Vero-B4 cell cultures

For addressing the question whether quinine shows a cytotoxic effect in the abovementioned systems non-infected Vero-B4 cells were treated in parallel to the Western blot and qRT-PCR studies with increasing concentrations of quinine sulfate (1 nM, 10 nM, 100 nM, 1 µM), 10 µM), 100 µM), 200 µM, 400 µM). Toxicity was assessed with a WST assay. Herein viable cells with an intact mitochondrial succinate-tetrazolium dehydrogenase system effect an enzymatic conversion of the feebly red tetrazolium salt WST-1 (4-[3-(4-iodophenyl)-2-(4-nitrophenyl)-2*H*-5-tetrazolio]-1,3 benzene disulfonate) into dark red formazan. This color change can be measured photometrically in a spectrophotometer. Thus, the WST assay is a very sensitive method for measuring the toxicity of substances on the cell metabolism.

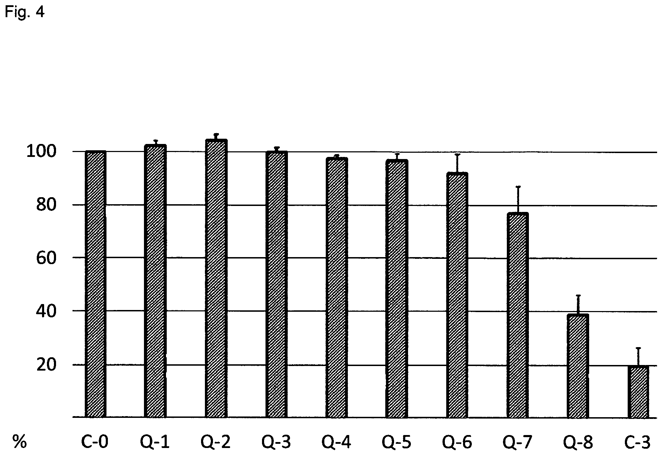

In Fig. 4 the percentage of viable cells is depicted in comparison to untreated cells. The value for untreated cells was set to 100%. 1 µM) staurosporine (an indolocarbazole compound from *Streptomyces staurosporeus,* an apoptosis inducer) was used as positive control.

It could be shown that quinine sulfate did not display any significant toxic effect in antivirally effective concentrations in Vero-B4 cells during an observation period of 3 days. Only at concentrations over 200 µM) a clear toxic effect was detectable.

Thus, it can be stated that the antiviral effect of quinine sulfate is not due to unspecific cytotoxic effects.

### Example 4: Quinidine sulfate inhibits dose-dependently the replication of SARS-CoV-2 in infected Vero-B4 cells in Western Blots (reference example)

In the same experimental setting as described in Example 1 it was tested whether quinidine sulfate is likewise able to reduce the viral replication. This time, the cells previously washed with PBS were provided with fresh medium containing 2.5, 5, 10 and 20 µM quinidine sulfate. Fresh medium alone served as negative control while chloroquine and hydroxychloroquine - 10µM each - served as positive controls. 10 µM quinine sulfate was used as additional control within the experiment.

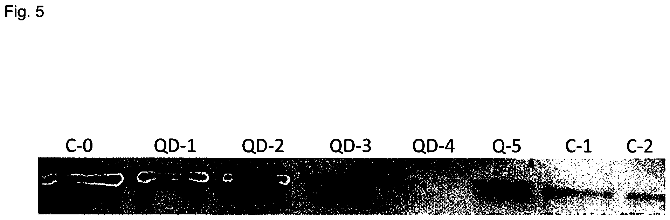

A clearly dose-dependent inhibition of SARS-Cov-2 replication was shown with a nearly complete block of virus replication at a concentration of 20 µM quinidine sulfate (Fig.5) and the reduction was clearly higher than after treatment with 10 µM chloroquine or 10 µM hydroxychloroquine.

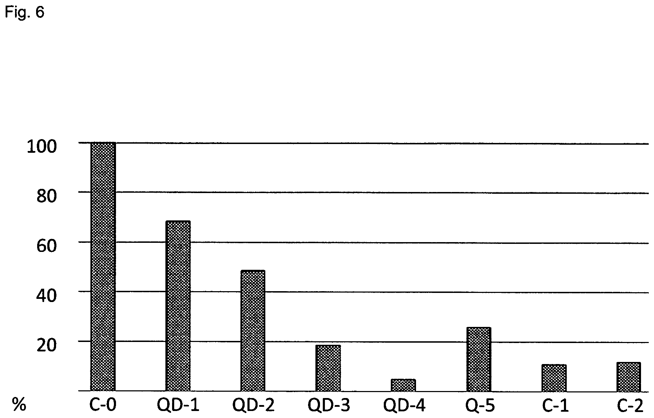

Densitometric evaluations of SARS-CoV-2 nucleoprotein in the virus fraction (AIDA^{®}) showed that also after the addition of quinidine sulfate the generation of SARS-CoV-2 proteins is inhibited in a dose-dependent manner and to a higher percentage as for the respective concentration of chloroquine or hydroxychloroquine (Fig. 6).

### Example 5: Quinidine sulfate inhibits the replication of SARS-CoV-2 in infected Vero-B4 cells in a dose-dependent manner in qRT-PCR (reference example)

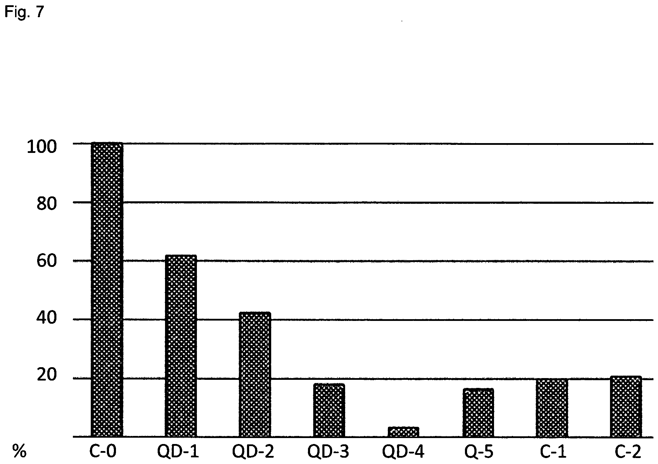

To evaluate the results of Example 4 qRT-PCR experiments were conducted following the same experimental setting as described in Example 2. This time the SARS-CoV-2 infected Vero-B4 cells were provided with medium containing quinidine sulfate in a non-cytotoxic concentration range (2.5 µM, 5 µM), 10 µM and 20 µM) resulting in a dose-dependent reduction of SARS-Cov-2 RNA copies (Fig. 7). At a concentration of 10 µM quinidine sulfate a reduction of approximately 82% and at 20 µM with approximately 97% a nearly complete block of virus spread was detected, the latter clearly providing a significant better reduction than after treatment with 10 µM chloroquine or 10 µM hydroxychloroquine. This confirms the results from Example 4.

### Example 6: In effective doses quinidine sulfate is not cytotoxic in Vero-B4 cell cultures (reference example)

To assess the cytotoxicity of quinidine sulfate shows a cytotoxic effect on non-infected Vero-B4 cells they were treated in parallel to the Western blot and qRT-PCR studies with increasing concentrations of quinidine sulfate (2.5 µM), 5 µM), 10 µM), 20 µM), 40 µM, 80 µM) following the same experimental setting as in Example 3.

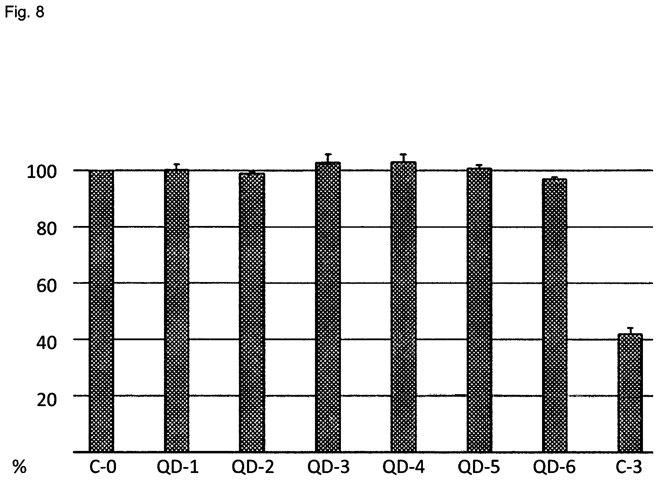

In Fig. 8 the percentage of viable cells is depicted in comparison to untreated cells wherein the value for untreated cells was set to 100%. Again 1 µM staurosporine was used as positive control.

It could be shown that quinidine sulfate did not display any significant toxic effect in antivirally effective concentrations in Vero-B4 cells during the 3 days observation period. Up to 80 µM no toxic effect was detectable at all.

Thus, it can be stated that the antiviral effect of quinidine sulfate is not due to unspecific cytotoxic effects.

### Example 7: Composition of a dietary supplement containing quinine hydrochloride

A dietary supplement drink is prepared by mixing the following substances in any order at room temperature under stirring:

| | |
|---|---|
| quinine hydrochloride | 25 mg |
| coenzyme Q10 | 50 mg |
| vitamin C | 20 mg |
| D-alpha tocopherol | 10 mg |
| aqua | ad 250 ml |

### Example 8: Composition of a dietary supplement containing quinine sulfate

A dietary supplement drink is prepared by mixing the following substances in any order at room temperature under stirring:

| | |
|---|---|
| quinine sulfate | 25 mg |
| zinc chloride | 20 mg |
| vitamin D | 20 µg |
| ascorbyl palmitate | 30 mg |
| aqua | ad 250 ml |

### FIGURES

- Fig. 1:: Western Blot bands of the Spike protein S1 of SARS-CoV-2 (trace A) and the Nucleoprotein of SARS-CoV-2 (trace B) after 3 days of treatment with quinine sulfate (Q) using concentrations of 100 nM (Q-3), 1 µM) (Q-4), 10 µM) (Q-5) and 100 µM (Q-6). Untreated cells served as negative control (C-0), Cells treated with 10 µM hydroxychloroquine (C-1) and 10 µM chloroquine (C-2) served as positive controls.
- Fig. 2:: Densitometric evaluation of Nucleoprotein of SARS-CoV-2 detected in Western Blot bands after 3 days of treatment with quinine sulfate (Q) using concentrations of 100 nM (Q-3), 1 µM (Q-4), 10 µM (Q-5) and 100 µM (Q-6). Untreated cells served as negative control (C-0), Cells treated with 10 µM hydroxychloroquine (C-1) and 10 µM chloroquine (C-2) served as positive controls. The percentage of the detected viral protein in treated vs. untreated cells is indicated. Untreated fraction was set to 100%.
- Fig. 3:: qRT-PCR analysis after 3 days of treatment with quinine sulfate (Q) using concentrations of 100 nM (Q-3), 1 µM (Q-4), 10 µM (Q-5) and 100 µM (Q-6). Untreated cells served as negative control (C-0), Cells treated with 10µM hydroxychloroquine (C-1) and 10 µM chloroquine (C-2) served as positive controls. The percentage of the detected RNA copies of SARS-CoV-2 in treated vs. untreated cells is indicated. Untreated fraction was set to 100%.
- Fig. 4:: Cell viability assessed in a WST-1 assay after addition of quinine sulfate (Q) in the concentrations 1 nM (Q-1), 10nM (Q-2), 100 nM (Q-3), 1 µM (Q-4), 10 µM (Q-5), 100 µM (Q-6), 200 µM (Q-7) and 400 µM (Q-8). The percentage of viable cells is indicated. Untreated cells (C-0) were taken as 100%. 1 µM staurosporine (C-3) was used as positive control. (mean ± SEM; n = 3)
- Fig. 5:: Western Blot bands of Nucleoprotein of SARS-CoV-2 after 3 days of treatment with quinidine sulfate (QD) using concentrations of 2.5 µM (QD-1), 5 µM (QD-2), 10 µM (QD-3) and 20 µM (QD-4), as well as with 10 µM quinine sulfate (Q-5). Untreated cells served as negative control (C-0). Cells treated with 10µM hydroxychloroquine (C-1) and 10 µM chloroquine (C-2) served as positive controls.
- Fig. 6:: Densitometric evaluation of Nucleoprotein detected in Western Blot bands after 3 days of treatment with quinidine sulfate (QD) using concentrations of 2.5 µM (QD-1), 5 µM (QD-2), 10 µM (QD-3) and 20 µM (QD-4), as well as with 10 µM of quinine sulfate (Q-5). Untreated cells served as negative control (C-0). Cells treated with 10µM hydroxychloroquine (C-1) and 10 µM chloroquine (C-2) served as positive controls. The percentage of the detected viral protein vs. untreated cells is indicated. Untreated fraction was set to 100%.
- Fig. 7:: qRT-PCR analysis after 3 days of treatment with quinidine sulfate (QD) using concentrations of 2.5 µM (QD-1), 5 µM (QD-2), 10 µM (QD-3) and 20 µM (QD-4), as well as with 10 µM of quinine sulfate (Q-5). Untreated cells served as negative control (C-0). Cells treated with 10µM hydroxychloroquine (C-1) and 10 µM chloroquine (C-2) served as positive controls. The percentage of the detected RNA copies of SARS-CoV-2 is indicated. Untreated fraction was set to 100%.
- Fig.8:: Cell viability assessed in a WST-1 assay after addition of quinidine sulfate (QD) in the concentrations 2.5 µM (QD-1), 5 µM (QD-2), 10 µM (QD-3), 20 µM (QD-4), 40 µM (QD-5) and 80 µM (QD-6) in the WST assay. The percentage of viable cells is indicated. Untreated cells (C-0) were taken as 100%. 1 µM of staurosporine (C-3) was used as positive control. (mean ± SEM; n = 3).

## Claims

1. Quinine or one of its pharmaceutically acceptable salts, hydrates and solvates for use by oral or pharyngeal administration in the prophylaxis or treatment of a coronaviral infection caused by SARS-CoV-2.

2. Quinine or one of its pharmaceutically acceptable salts, hydrates and solvates for use according to Claim 1, wherein the oral administration is effected by sublingual tablets.

3. Quinine or one of its pharmaceutically acceptable salts, hydrates and solvates for use according to Claim 1, wherein the administration is effected by pharyngeal administration.

4. Quinine or one of its pharmaceutically acceptable salts, hydrates and solvates for use according to Claim 3, wherein the pharyngeal administration is carried out by means of a throat spray.

## Patentansprüche

1. Chinin oder eines seiner pharmazeutisch verträglichen Salze, Hydrate und Solvate zur Verwendung bei oraler oder pharyngealer Verabreichung zur Prophylaxe oder Behandlung einer durch SARS-CoV-2 verursachten coronaviralen Infektion.

2. Chinin oder eines seiner pharmazeutisch verträglichen Salze, Hydrate und Solvate zur Verwendung gemäß Anspruch 1, wobei die orale Verabreichung durch sublinguale Tabletten erfolgt.

3. Chinin oder eines seiner pharmazeutisch verträglichen Salze, Hydrate und Solvate zur Verwendung gemäß Anspruch 1, wobei die Verabreichung über eine pharyngeale Verabreichung erfolgt.

4. Chinin oder eines seiner pharmazeutisch verträglichen Salze, Hydrate und Solvate zur Verwendung gemäß Anspruch 3, wobei die pharyngeale Verabreichung mittels eines Rachensprays ausgeführt wird.

## Revendications

1. Quinine ou l'un de ses sels hydrates ou solvates pharmaceutiquement acceptables, pour une utilisation en administration orale ou pharyngée pour la prophylaxie ou le traitement d'une infection coronavirale causée par SARS-CoV-2.

2. Quinine ou l'un de ses sels hydrates ou solvates pharmaceutiquement acceptables, pour une utilisation selon la revendication 1, dans laquelle l'administration orale est effectuée par des comprimés sublinguaux.

3. Quinine ou l'un de ses sels hydrates ou solvates pharmaceutiquement acceptables, pour une utilisation selon la revendication 1, dans laquelle l'administration est effectuée par une administration pharyngée.

4. Quinine ou un de ses sels, hydrates et solvates pharmaceutiquement acceptables pour une utilisation selon la revendication 3, dans laquelle l'administration pharyngée est effectuée au moyen d'un spray pharyngé.
